# EUROPEAN PATENT SPECIFICATION

(11) **EP 2 317 991 B1**
(45) Date of publication and mention of the grant of the patent: **03.05.2017**
(21) Application number: 09780282.1
(22) Date of filing: 07.07.2009
(51) Int. Cl.: A61K 31/00, A61K 31/485, A61P 13/02

(54) **USE OF OPIOID ANTAGONISTS FOR TREATING URINARY RETENTION**
VERWENDUNG VON OPIOID-ANTAGONISTEN ZUR BEHANDLUNG VON HARNVERHALT
UTILISATION D'ANTAGONISTES OPIOÏDES POUR LE TRAITEMENT D'UNE RÉTENTION URINAIRE

(30) Priority: 07.07.2008 EP 08159802
(43) Date of publication of application: 11.05.2011
(73) Proprietor: EURO-CELTIQUE S.A., 2350 Luxembourg (LU)
(72) Inventor: HOPP, Michael, 65520 Bad Camberg (DE); LEYENDECKER, Petra, 35619 Braunfels (DE)
(74) Representative: Maiwald Patentanwalts GmbH
(86) International application number: PCT/EP2009/058630
(87) International publication number: WO 2010/003963

(56) References cited:
- EP-A- 1 695 700
- ROSOW C E ET AL: "Reversal of opioid-induced bladder dysfunction by intravenous naloxone and methylnaltrexone" CLINICAL PHARMACOLOGY & THERAPEUTICS, MOSBY-YEAR BOOK, ST LOUIS, MO, US, vol. 82, no. 1, 1 July 2007 (2007-07-01), pages 48-53, XP009123214 ISSN: 0009-9236
- SANDNER F: "Hoffnung für Schmerzpatienten: Fixkombination aus Naloxon und Oxycodon bietet Analgesie und Obstipationsschutz auch im Schlaf = Hope for patients with chronic pain: Naloxone and oxycodone fixed combination offers analgesia and prevention of constipation also in sleep" JOURNAL FÜR PHARMAKOLOGIE UND THERAPIE = JOURNAL OF PHARMACOLOGY AND THERAPY, PIA : PERFUSION, DE, vol. 16, no. 6, 1 June 2007 (2007-06-01), pages 179-180, XP009123174 ISSN: 1432-4334
- Anonymous: "Targin®: Oxycodon und Naloxon als Fixkombination bei Schmerztherapie, 2006", , 1 January 2006 (2006-01-01), Retrieved from the Internet: URL:http://www.medknowledge.de/neu/med/jah r/2006/IV-2006-56-targin.htm [retrieved on 2014-01-21]
- Peter Holzer: "Opioid receptors in the gastrointestinal tract", REGULATORY PEPTIDES., vol. 155, no. 1-3, 1 June 2009 (2009-06-01), pages 11-17, XP055260065, NL ISSN: 0167-0115, DOI: 10.1016/j.regpep.2009.03.012

## Description

### TECHNICAL FIELD OF THE INVENTION

The invention pertains to at least one opioid antagonist and at least one opioid agonist for use in the treatment of urinary retention as claimed herein.

### BACKGROUND OF THE INVENTION

Urinary retention is a condition by which a substantial number of patients are affected and which results from different, often unrelated underlying causes.

It is defined by symptoms of decreased urinary stream with intermittence, straining, urgency and incomplete voiding. Acute and chronic retention has to be differentiated.

Urinary retention can cause urinary urge or overflow incontinence. It may cause abdominal distention and pain. Long-term urinary retention predisposes to urinary tract infections (UTI) and can increase bladder pressure, causing obstructive uropathy. Consequences of chronic urinary retention thus can include development of bladder stones, loss of detrusor muscle tone, hydronephrosis, hypertrophy of detrusor muscle and diverticula in the bladder wall. Particularly acute urinary retention can be very painful.

Several reasons, diseases, conditions can act as basis for development of urinary retention. This can be e.g. benign prostatic hypertrophiy, prostatic cancer or other pelvic cancer, congenital abnormalities like urethral valve abnormalities, detrusor muscle dyssynergia, circumcision, damage to the bladder, obstruction in the urethra, paruresis, faecal impaction, acute or chronic prostatitis, blood clots in the bladder, retroverted gravid uterus, spinal anaesthesia or postoperative conditions, spinal cord injury, urethral rupture, anal pain, ureter stones. Drugs like anticholinergics, antidepressants, opioids can also cause urinary retention due to the effect of the neuronal system and smooth muscles.

Urinary retention typically occurs although the kidney function may be normal, as urine will be produced but cannot be excreted. The therapeutic aim therefore usually is not to increase the production of urine but to ease excretion thereof.

Treatment for acute and chronic urinary retention may differ. In acute urinary retention, intravesicale catheterization via the urethra or suprapubic cystotomie may be the first therapeutic intervention. For chronic urinary retention being e.g. based on prostate hypertrophy, pharmaceutical treatments such as alpha reductase inhibitors or an operational ectomisation of the prostate gland via open prostatectomy or transurethral resection (TURP) may be first choice.

Although these principles are established as general treatment routes, therapy options may differ and have to be adapted dependent on the different pathophysiological causes for urinary retention. As mentioned, pharmaceuticals which are used in urinary retention are e.g. alpha 1 receptor blockers (doxazosin, prazosin, phenoxybenzamine, phentolamine, tamsulosin, alfuzosin and terazosin) or 5 alpha reductase inhibitors (finasteride, dutasteride). There is no specific treatment for opioid induced urinary retention available based on oral dosage forms.

It is noted that Rosow et al. (Clinical Pharmaceology & Therapeutics, MOSBY-YEAR BOOK, ST LOUIS, MO, US, vol. 82, no. 1, 1 July 2007 pages 48-53) discloses intravenously administered methylnaltrexone and naloxone for reversing opioid-induced bladder dysfunction. It is further noted that EP 1 695 700 A1 and Sandner F (Journal für Pharmakologie und Therapie, PIA: Perfusion, DE, vol. 16, no. 6, 1 June 2007, pages 179-180) disclose pharmaceutical compositions comprising naloxone and oxycodone for the treatment of pain and the alleviation of the opioid-induced side effect constipation.

There is nevertheless a continuing need for additional pharmaceutical dosage forms and treatment regimens.

### OBJECT AND SUMMARY OF THE INVENTION

It is one objective of the present invention to provide new pharmaceutical dosage forms that allow treatment of urinary retention in human beings.

Further, it is an objective to provide known pharmaceutical preparations for their novel use of treating urinary retention.

These and other objectives as they will become apparent from the ensuing description are attained by the subject matter of the independent claims. Some of the preferred embodiments of the inventions are the subject matter of the dependent claims.

In one embodiment the present invention pertains to a pharmaceutical composition comprising at least one opioid antagonist or a pharmaceutically acceptable salt thereof and at least one opioid agonist or a pharmaceutically acceptable salt thereof for use in treating urinary retention as claimed in independent claim 1.

The opioid antagonist is naloxone or a pharmaceutically acceptable salt thereof. A preferred pharmaceutically acceptable salt of naloxone is naloxone hydrochloride.

A particularly preferred embodiment relates to a pharmaceutical composition comprising naloxone or a pharmaceutically acceptable salt thereof such as the hydrochloride salt as the sole opioid antagonist.

The pharmaceutical compositions in accordance with the invention may be used for treating non-opioid induced urinary retention.

Pharmaceutical compositions in accordance with the invention may release the opioid antagonist or a pharmaceutically acceptable salt thereof and the at least one opioid agonist or a pharmaceutically acceptable salt thereof immediately or in a controlled manner.

A controlled release pharmaceutical composition as claimed comprising at least one opioid antagonist or a pharmaceutically acceptable salt thereof may comprise up to about 30% of the at least one opioid antagonist or a pharmaceutically acceptable salt thereof being formulated for immediate release and may nevertheless be classified as a controlled release dosage form.

Controlled release pharmaceutical compositions as claimed comprising at least one opioid antagonist or a pharmaceutically acceptable salt thereof may comprise a matrix and/or a coating for providing controlled release properties.

Pharmaceutical compositions in accordance with the invention comprise in addition to the at least one opioid antagonist or pharmaceutically acceptable salt thereof at least one opioid analgesics or pharmaceutically acceptable salts thereof, in particular if the urinary retention from which the patient suffers causes moderate to severe pain. Opioid analgesics are preferably selected from the group comprising oxycodone, morphine, hydromorphone, oxymorphone, buprenorphine, noroxymorphone, tramadol or the like.

A preferred additional pharmaceutically active agent is oxycodone. A preferred pharmaceutically acceptable salt thereof is oxycodone hydrochloride.

The dosage forms in accordance with the present invention in a preferred embodiment comprise naloxone hydrochloride and oxycodone hydrochloride in a 1:2 ratio by weight. Preferably the dosage forms comprise the aforementioned active agents as the sole pharmaceutically active agents.

The pharmaceutical compositions in accordance with the invention are formulated for oral application. They may thus take the form of a liquid, a tablet, a pill, a capsule, a granule, a spheroid etc. The pharmaceutical dosage forms may be multiparticulate dosage forms.

Another embodiment of the present invention relates to an oral pharmaceutical composition comprising at least oxycodone or a pharmaceutically acceptable salt thereof and naloxone or a pharmaceutically acceptable salt thereof for use in treating pain in patients which have to discontinue opioid-based pain therapy for development of urinary retention, wherein said composition is a controlled release dosage form.

Preferably such pharmaceutical compositions comprise oxycodone hydrochloride and naloxone hydrochloride.

In one embodiment such pharmaceutical compositions comprise oxycodone hydrochloride and naloxone hydrochloride in a 2:1 ratio by weight.

The compositions may comprise oxycodone or a pharmaceutically acceptable salt thereof such as the hydrochloride salt and naloxone or a pharmaceutically acceptable salt thereof such as the hydrochloride salt as the sole pharmaceutically active agents.

### DETAILED DESCRIPTION OF THE INVENTION

The inventors of the invention have found that the oral administration of naloxone in the form of a controlled release composition comprising the active agents oxycodone hydrochloride and naloxone hydrochloride leads to a significant reduction of opioid induced urinary retention. Based on these findings, the present invention is directed at naloxoneor a pharmaceutically acceptable salt thereof and at least one opioid agonist or a pharmaceutically acceptable salt thereof for use in treating urinary retention including non-opioid induced urinary retention in human patients.

Before specific aspects and some of the preferred embodiments as mentioned above are described in further detail, the following definitions are provided which shall have the indicated meaning throughout the description of the invention, unless explicitly indicated otherwise by the respective context.

The present invention will be described with respect to particular embodiments but the invention is not limited thereto but only by the claims.

Where the term "comprising" is used in the present description and claims, it does not exclude other elements. For the purposes of the present invention, the term "consisting of" is to be a preferred embodiment of the term "comprising". If hereinafter a group is defined to comprise at least a certain number of embodiments, this is also to be understood to disclose a group that preferably consists only of these embodiments.

Where an indefinite or definite article is used when referring to a singular noun, i.e. "a", "an", or "the", this includes a plural of that noun unless something else is specifically stated. The terms "about" and "approximately" in the context of the present invention denote an interval of accuracy that the person skilled in the art will understand to still ensure the technical effect of the feature in question. The term typically indicates the deviation from an indicated numerical value of ±10%, and preferably of ±5%.

As already mentioned above the present invention relates to pharmaceutical compositions comprising at least one opioid antagonist or a pharmaceutically acceptable salt thereof and at least one opioid agonist or a pharmaceutically acceptable salt thereof for use in treating urinary retention as claimed in independent claim 1.

According to the present invention "opioid antagonists" comprise such compounds that counteract the effect of opioid agonists, the latter also being designated as opioid analgesics. Such compounds can also be found in the ATC classification of the WHO. Opioid antagonists may be selected from the group comprising naloxone, naltrexone, nalmemefen, nalorphene, nalbuphene, naloxoneazenen, methyl naltrexone, ketylcyclazocene, norbenaltorphinene, naltrendol, 6-β-naloxole and 6-β-naltroxone. The opioid antagonist of the present invention is naloxone.

The opioid antagonist or any of the other pharmaceutically active agents mentioned hereinafter may be present in the pharmaceutical dosage forms of the present invention as free base. However, an opioid antagonist or any of the other pharmaceutically active agents mentioned hereinafter may also be present in the form of its pharmaceutically acceptable salts. Such salts include e.g. the hydrochloride salt, the sulphate salt, the bisulphate salt, the tartrate salt, the nitrate salt, the citrate salt, the bitartrate salt, the phosphate salt, the malate salt, the maleate salt, the hydrobromide salt, the hydroiodide salt, the fumarate salt, the succinate salt and the like. A pharmaceutically active agent as mentioned hereinafter may also be present as base addition salts such as metal salt of alkali metals including lithium, sodium and potassium.

The pharmaceutical dosage forms may comprise additional pharmaceutically acceptable excipients. These pharmaceutical excipients as well as the manufacturing methods may be chosen so as to convey certain release properties to the pharmaceutical dosage forms in accordance with the invention. Dependent on the release characteristics, the pharmaceutical dosage forms in accordance with the invention may be classified as immediate release dosage forms or controlled release dosage forms.

The term "immediate release dosage form" is typically used to describe a pharmaceutical dosage form that releases about 70% by weight of the pharmaceutically active agent, such as an opioid antagonist or pharmaceutically acceptable salt thereof within 30 minutes after administration. The release is typically measured using the European Pharmacopoeia Paddle Test at 50 rpm in 900 ml 0.1 N HCl pH 1.2 using UV detection at 270 nm.

The term "controlled release dosage form" is typically used to highlight that a pharmaceutical dosage form is not an immediate release (IR) pharmaceutical dosage form, but releases the active agent(s) from the dosage form over longer periods of time than mentioned above such that a prolonged therapeutic activity is achieved. In this case, controlled release dosage forms are also designated as "sustained release dosage forms".

The terms "controlled release" and "sustained release" refer thus to the release of a pharmaceutically active compound from a dosage form over an extended period of time. In general, controlled release dosage forms in the context of the present invention means that the opioid antagonist or a pharmaceutically acceptable salt thereof and/or additional pharmaceutically active agents or their pharmaceutically acceptable salts as mentioned herein are released from the pharmaceutical dosage form over a time period of at least 4 hours. Preferably, the release of the pharmaceutically active agents from the dosage form may take place over time periods of at least 6 hours, at least 8 hours, at least 10 hours, at least 12 hours or at least 14 hours.

The European Pharmacopoeia Paddle Test as mentioned above can be used to determine the release of the active agent(s). Reference to the European Pharmacopoeia Paddle Test in the context of *in vitro* release data refers only to the method of measurement but not in any way to evaluating the measured data even if such evaluation approaches are mentioned in the Pharmacopoeia. Thus, the indicated release values do not relate to average values of such measurements unless indicated otherwise.

The controlled release pharmaceutical dosage forms in accordance with the invention may release the pharmaceutically active agent over prolonged periods of time such that the dosage forms can be administered at reduced frequency compared to immediate release dosage forms. Depending on the frequency of administration, controlled release pharmaceutical preparations in accordance with the invention can be classified as three times a day, twice a day or once a day dosage forms. Twice a day or once a day dosage forms are preferred.

It is to be understood that the term "controlled release pharmaceutical dosage form" refers to a dosage form as such. Thus, the classification of a dosage form as providing controlled release is to be decided on the basis of the *in vitro* release data of the complete dosage form. Therefore, if a dosage form releases the active agent(s) over an extended period of time as mentioned above and described in further detail hereinafter, it will be considered to be a controlled release dosage form even if it comprises in addition a portion of pharmaceutically active agent(s) that has been formulated to provide immediate release characteristics. Typically, the amount of pharmaceutically active agent within a dosage form that may be formulated for immediate release can be up to 30% with the overall dosage form still being classified as a controlled release dosage form. Such immediate release phases may allow fast achievement of a therapeutic effect while the controlled release portion will ensure that the active agents are released over a prolonged period of time so that the therapeutic effect is maintained over a time longer than it would be for a purely immediate release dosage form.

Controlled release characteristics can be achieved by different formulation approaches.

For example, a pharmaceutical dosage form may comprise a controlled release matrix in which the pharmaceutically active agent is embedded in order to achieve the sustained release properties of the dosage form.

In another embodiment, a coating approach may be used to ensure the controlled release characteristics of a pharmaceutical dosage form.

These approaches for achieving controlled release of active agent, i.e. use of a matrix or use of a coating may of course also be combined. The person skilled in the art is further aware of other technical approaches for achieving a sustained release of the dosage form that include e.g. osmotically driven controlled release dosage form.

In a preferred embodiment, the present invention contemplates pharmaceutical dosage forms as claimed in independent claim 1 that comprise a matrix for achieving controlled release of the antagonist or the pharmaceutically acceptable salt thereof and the agonist or the pharmaceutically acceptable salt thereof. To this end, the controlled release matrix may comprise a substantial amount of the opioid antagonist and the opioid agonist that are present within the dosage form. Typically, if a controlled release matrix system is used, the pharmaceutically active agents such as the opioid antagonist and opioid agonist will be dispersed throughout a matrix-forming material.

The matrix-forming materials may be chosen to achieve an erosive matrix, a diffusion matrix or a matrix system which combines the characteristic of an erosive and a diffusion matrix. Suitable materials for inclusion in a controlled release matrix include:
(a) Hydrophilic or hydrophobic polymers, such as gums, cellulose ethers, acrylic resins and protein derived materials. Of these polymers, the cellulose ethers, especially alkylcelluloses are preferred. The preparation may conveniently contain between 1% and 80% (by weight) of one or more hydrophilic or hydrophobic polymers.
(b) Digestible, long chain (C₈-C₅₀, especially C₁₂-C₄₀), substituted or unsubstituted hydrocarbons, such as fatty acids, fatty alcohols, glycerol esters of fatty acids, mineral and vegetable oils and waxes. Hydrocarbons having a melting point of between 25 and 90°C are preferred. Of these long chain hydrocarbon materials, fatty (aliphatic) alcohols are preferred. The preparation may conveniently contain up to 60% (by weight) of at least one digestible, long chain hydrocarbon.
(c) Polyalkylene glycols. The preparation may suitably contain up to 60% (by weight) of one or more polyalkylene glycols.

In a preferred embodiment, the pharmaceutical dosage forms as claimed herein will use a diffusion matrix for achieving sustained release of the opioid antagonist or its pharmaceutically acceptable salt and the opioid agonist or its pharmaceutically acceptable salt from the pharmaceutical dosage form.

To this end, the diffusion matrix may be made from a hydrophobic polymer and/or a C₁₂-C₃₆ fatty alcohol.

As regards the hydrophobic polymer, use of a hydrophobic cellulose ether and particularly ethyl cellulose may be preferred.

As regards the fatty alcohol, use of lauryl, myristyl, stearyl, cetylstearyl, ceryl and/or cetylalcohol will be preferably considered. The use of stearyl alcohol is particularly preferred.

A particularly preferred embodiment relates to pharmaceutical dosage forms as claimed in independent claim 1 in which the controlled release properties of the opioid antagonist or the pharmaceutically acceptable salt thereof and the opioid agonist or the pharmaceutically acceptable salt thereof are provided by a diffusion matrix which is made from a hydrophobic polymer such as from ethyl cellulose and a fatty alcohol. The matrices of some of the preferred embodiments of the invention, which may e.g. be made from the aforementioned combination of ethyl cellulose and stearyl alcohol, will be substantially non-swellable diffusion matrix.

The term "substantially non-swellable diffusion matrix" indicates that the matrix will be substantially non-erosive, i.e. that the size of the matrix will not significantly increase upon contact with fluids. Typically, the volume of a substantially non-swellable diffusion matrix will increase at maximum up to 100 %, preferably at maximum up to 75 %, more preferably at maximum up to 50 %, even more preferably at maximum up to 25% and most preferably at maximum up to 10 % or at maximum up to 5 % in volume upon contacting an aqueous solution.

Pharmaceutical dosage forms which comprise a hydrophobic polymer with hydrophobic cellulose ethers such as ethyl cellulose being preferred as the sole or one of the components for providing a controlled release (non-swellable) diffusion matrix, will use an amount of such polymer of between 5 to 20%, preferably of between 6 and 15% by weight and more preferably of between 7 to 10% by weight. The percentages indicate the amount of the matrix-forming material with respect to the total weight of the pharmaceutical dosage form.

Pharmaceutical dosage forms, which comprise a fatty alcohol as the sole or one of the components for providing a controlled release diffusion matrix, will use an amount of fatty alcohol in the matrix of between 10 to 40%, preferably of between 15 to 35 % and more preferably of between 17 to 25% by weight. These percentages again indicate the amount of fatty alcohol based on the total weight of the dosage form.

The person skilled in the art is further aware that such a controlled release matrix may also contain other pharmaceutically acceptable ingredients and excipients which are conventional in the pharmaceutical art such as diluents, lubricants, fillers, binders, flowing agents, colourants, flavourants, surfactants, pH-adjusters, anti-tacking agents. These excipients will typically have no substantial impact on the overall release behaviour of the pharmaceutical dosage form.

Typical examples of fillers comprise lactose, glucose, saccharose, starch and their hydrolysates, microcrystalline cellulose, calcium salts like calcium hydrogen phosphate etc. Granulating aids comprise *inter alia* povidone. Flowing agents and lubricants comprise *inter alia* highly dispersed silica, talcum, magnesium oxide and magnesium stearate. Matrix-based dosage form may e.g. comprise a cosmetic coating.

As mentioned above, controlled release characteristics of a pharmaceutical dosage form may also be achieved by a film coating that governs the release of the active agents from the dosage form. To this end, the pharmaceutical dosage form may comprise a carrier, which is associated with the opioid antagonist or the pharmaceutically acceptable salts thereof. For example, one may use nonpareil beads, sugar beads etc. on and/or into which the pharmaceutically active agents are disposed.

Such active-associated carriers may then be overcoated with a coating that provides controlled release characteristics. Suitable controlled release coating materials include hydrophobic polymers such as cellulose ethers and/or acrylic polymer resins. Ethylcellulose may be preferred.

The controlled release coatings may comprise other components such as hydrophilic substances including hydrophilic polymers such hydroxypropylmethylcellulose (HPMC), polyethylenglycols etc. These components may be used to adjust the controlled release characteristics of the coatings. In case of e.g. HPMC, the substances may act as pore formers. The coating may, of course, also comprise additional pharmaceutically acceptable excipients.

Controlled release dosage forms that use a coating for providing prolonged release may comprise an immediate release phase of the active above the coating and typically still be classified as controlled release dosage form.

Further, the person skilled in the art will realize that not only controlled release matrices and coatings as mentioned above, but also the pharmaceutical dosage forms as a whole may also comprise additionally the above mentioned pharmaceutically acceptable excipients.

As mentioned above, the pharmaceutical dosage forms in accordance with the invention can be used for treating urinary retention. Urinary retention may result from different, and in some cases unrelated underlying reasons. In one embodiment, the pharmaceutical dosage forms in accordance with the invention are used for the treatment of non-opioid induced urinary retention.

Urinary retention may result from bladder dysfunction and other underlying causes such as mentioned in the background section.

As mentioned above, the opioid antagonist of the present invention is naloxone. A preferred pharmaceutically acceptable salt of an opioid antagonist to be used in preparations in accordance with the invention is naloxone hydrochloride.

Typically, naloxone or its pharmaceutically acceptable salt may be used in amounts in the dosage forms equivalent to about 1 to about 80 mg, about 1 to about 40 mg, preferably about 2 mg, about 4 mg, about 6 mg, about 8 mg, about 10 mg, about 15 mg, about 20 mg, about 25 mg or about 30 mg naloxone hydrochloride. These amounts refer to the amount of naloxone or a pharmaceutically acceptable salt thereof such as naloxone hydrochloride in the dosage form. As the dosage forms may be administered in multiples, the administered amount of naloxone or its salts may be higher.

Preferably the dosage forms in accordance with the invention will be a controlled release dosage form and is formulated for oral administration.

A particularly preferred embodiment will relate to controlled release pharmaceutical preparations for treating urinary retention which comprise 1 to 40 mg of naloxone hydrochloride per unit dose and which are formulated for oral administration.

The present invention in one of its most preferred embodiments relates to a controlled release oral pharmaceutical dosage form as claimed in independent claim 1 for use in treating urinary retention wherein the pharmaceutical dosage form comprises naloxone or a pharmaceutically acceptable salt thereof in an amount equivalent to 2.5 to 20 mg of naloxone hydrochloride per unit dose, and wherein the controlled release dosage form releases naloxone or a pharmaceutically acceptable salt thereof by the following *in vitro* dissolution rates when measured according to the European Pharmacopeia paddle test at 50 rpm in 900 ml 0.1 N HCl pH 1.2 using UV detection at 270 nm:
10 - 30 % by weight of naloxone or said salt thereof at 15 min,
30 - 50 % by weight of naloxone or said salt thereof at 1 h,
45 - 65 % by weight of naloxone or said salt thereof at 2 h,
60 - 85 % by weight of naloxone or said salt thereof at 4 h,
70 - 95 % by weight of naloxone or said salt thereof at 7 h, and
≥ 80% by weight of naloxone or said salt thereof at 10 h.

In another embodiment the pharmaceutical dosage forms as claimed in independent claim 1 may display the following *in vitro* dissolution rates when measured according to the United States Pharmacopoeia Basket Method at pH 1.2 using UV detection at 270 nm:
15 to 30 % by weight of naloxone or said salt thereof at 15 min,
45 to 70% by weight of naloxone or said salt thereof at 2 h,
≥ 80% by weight of naloxone or said salt thereof at 7 h,
≥ 90% by weight of naloxone or said salt thereof at 12 h.

The term "opioid agonist" is used as known in the art. For the purposes of the present invention it will be considered to be equivalent to the term "opioid analgesic". Typically, a pharmaceutically active agent will be considered to be an opioid analgesic or opioid agonist if it belongs to Class NO2A of opioid analgesics according to the Anatomical Therapeutic Chemical classification (ATC classification) of the World Health Organisation (WHO). Preferably, an opioid agonist may be selected from the group comprising morphine, oxycodone, hydromorphone, propoxyphene, nicomorphine, dihydrocodeine, diamorphine, papaveretum, codeine, ethylmorphine, phenylpiperidine and derivates thereof, methadone, dextropropoxyphene, buprenorphine, pentazocine, tilidine, tramadol, hydrocodone. Further examples for useable analgesics according to the invention are meperidine, oxymorphone, alphaprodine, anileridine, dextromoramide, metopone, levorphanol, phenazocine, etoheptazine, propiram, profadol, phenampromide, thiambuten, pholcodeine, codeine, dihydrocodeine, fentanyl, 3-trans-dimethylamino-4-phenyl-4-trans-carbethoxy-A'-cyclohexen, 3-dimethylamino-0-(4-methoxyphenyl-carbamoyl)-propiophenone oxime, (-)β-2'-hydroxy-2, 9-dimethyl-5-phenyl-6, 7-benzomorphane, (-)2'-hydroxy-2-(3-methyl-2-butenyl)-9-methyl-5-phenyl-6, 7-benzomorphane, pirinitramide, (-)α-5,9-diethyl-2' hydroxy-2-methyl-6, 7-benzomorphane, ethyl 1-(2-dimethylaminoethyl)-4,5,6,7-tetrahydro-3-methyl-4-oxo-6-phenyl-indol-2-carboxylate, 1-benzoylmethyl-2, 3-dimethyl-3- (m-hydroxyphenyl) -piperidine, N-allyl-7α (1-R-hydroxy-1-methylbutyl)-6,14-endo-ethanotetrahydronororipavine, (-)2'-hydroxy-2-methyl-6,7-benzomorphane, noracylmethadol, phenoperidine, α-d1-methadol, α -1-methadol, β-d1-acetylmethadol, α-1-acetylmethadol and β-1-acetylmethadol. Preferred opioid agonists according to the present invention are oxycodone, hydrocodone, hydromorphone, morphine, codeine, dihydrocodeine, oxymorphone and fentanyl. The opioid agonist oxycodone can be particularly preferred.

A particularly preferred pharmaceutically acceptable salt is oxycodone hydrochloride.

The person skilled in the art will be aware of selecting an amount of opioid agonist and to select the ratio of opioid agonist and antagonist such that the opioid agonist will be capable of treating pain resulting from urinary retention while avoiding that urinary retention results as a side effect from the opioid treatment.

Typically, if oxycodone is used as an opioid agonist, it will be included in an amount being equivalent to 5, 10, 20, 40, 60, 80, 100, 120 or 160 mg oxycodone hydrochloride per unit dose.

If the opioid antagonist is naloxone hydrochloride and the opioid agonist is oxycodone hydrochloride, these pharmaceutically active agents may be combined in a 1:2 ratio by weight of naloxone hydrochloride to oxycodone hydrochloride. In such cases pharmaceutical dosage forms in accordance with the invention may comprise oxycodone in an amount up to and being equivalent to 5, 10, 20, 40, 80, 100, 120 or 160 mg of oxycodone hydrochloride per unit dose and naloxone in an amount up to and being equivalent to 2.5, 5, 10, 20, 40, 50 or 80 mg of naloxone hydrochloride per unit dose. These dosage forms may preferably be controlled release dosage forms and are formulated for oral administration.

The present invention in one of its most preferred embodiments relates to a controlled release oral pharmaceutical dosage form for use in treating urinary retention and the pain caused thereby wherein the pharmaceutical dosage form comprises oxycodone hydrochloride and naloxone hydrochloride in a 2:1 weight ratio with oxycodone hydrochloride being present in an amount of 10 to 40 mg per unit dose and naloxone hydrochloride being present in an amount of 5 to 20 mg per unit dose.

Preferably such controlled release dosage forms release oxycodone hydrochloride and naloxone hydrochloride by the following *in vitro* dissolution rates when measured according to the European Pharmacopeia paddle test at 50 rpm in 900 ml 0.1 N HCl pH 1.2 using UV detection at 270 nm:
10 - 30 % by weight of oxycodone hydrochloride at 15 min,
30 - 50 % by weight of oxycodone hydrochloride at 1 h,
40 - 65 % by weight of oxycodone hydrochloride at 2 h,
60 - 85 % by weight of oxycodone hydrochloride at 4 h,
70 - 95 % by weight of oxycodone hydrochloride at 7 h,
≥ 80% by weight of oxycodone hydrochloride at 10 h,
10 - 30 % by weight of naloxone hydrochloride at 15 min,
30 - 50 % by weight of naloxone hydrochloride at 1 h,
45 - 65 % by weight of naloxone hydrochloride at 2 h,
60 - 85 % by weight of naloxone hydrochloride at 4 h,
70 - 95 % by weight of naloxone hydrochloride at 7 h, and
≥ 80% by weight of naloxone hydrochloride at 10 h.

In another embodiment the pharmaceutical dosage forms may display the following *in vitro* dissolution rates when measured according to the United States Pharmacopoeia Basket Method at pH 1.2 using UV detection at 270 nm:
15 to 30 % by weight of oxycodone hydrochloride at 15 min,
45 to 70% by weight of oxycodone hydrochloride at 2 h,
≥ 80% by weight of oxycodone hydrochloride at 7 h,
≥ 90% by weight of oxycodone hydrochloride at 12 h,
15 to 30 % by weight of naloxone hydrochloride at 15 min,
45 to 70% by weight of naloxone hydrochloride at 2 h,
≥ 80% by weight of naloxone hydrochloride at 7 h,
≥ 90% by weight of naloxone hydrochloride at 12 h.

Another embodiment of the present invention relates to an oral pharmaceutical composition comprising at least oxycodone or a pharmaceutically acceptable salt thereof and naloxone or a pharmaceutically acceptable salt thereof for treatment of pain in patients which otherwise have to discontinue opioid-based pain therapy for development of urinary retention wherein said composition is an oral controlled release dosage form.

It has been observed that opioid-based treatment of moderate to severe pain can cause urinary retention as a side effect. In some patients, the impact of this side effect can reach a degree that opioid therapy has to be discontinued.

However, by combining the opioid agonist oxycodone and the opioid antagonist naloxone or their pharmaceutically acceptable salts, it is possible to treat patients suffering from pain which otherwise have to discontinue a pain treatment that is based on an opioid agonist as the sole pharmaceutically active agent. Therefore, such pharmaceutical dosage forms can particularly be used to treat patients such as the elderly which suffer from impaired activity of the urinary tract system.

Preferably such pharmaceutical compositions comprise oxycodone hydrochloride and naloxone hydrochloride.

In one embodiment such pharmaceutical compositions comprise oxycodone hydrochloride and naloxone hydrochloride in a 2:1 ratio by weight.

Such preparations are preferably used for treatment of moderate to severe pain.

The compositions may comprise oxycodone or a pharmaceutically acceptable salt thereof such as the hydrochloride salt and naloxone or a pharmaceutically acceptable salt thereof such as the hydrochloride salt as the sole pharmaceutically active agents.

The preparations are controlled release preparations and may be based on a controlled release matrix, a controlled release coating or other controlled release functionalities. As regards the structure, composition, excipients, *in vitro* release data, amount of active agents etc. of such controlled release preparations comprising oxycodone and naloxone, reference is made to the passages mentioned before.

Typically, if oxycodone is used as an opioid analgesic as the additional pharmaceutically active agent, it will be included in an amount being equivalent to 5, 10, 20, 40, 60, 80, 100, 120 or 160 mg oxycodone hydrochloride per unit dose.

If the opioid antagonist is naloxone hydrochloride and the opioid agonist is oxycodone hydrochloride, these pharmaceutically active agents may be combined in a 1:2 ratio by weight of naloxone hydrochloride to oxycodone hydrochloride. In such cases pharmaceutical dosage forms in accordance with the invention may comprise oxycodone in an amount up to and being equivalent to 5, 10, 20, 40, 80, 100, 120 or 160 mg of oxycodone hydrochloride per unit dose and naloxone in an amount up to and being equivalent to 2.5, 5, 10, 20, 40, 50, 60 or 80 mg of naloxone hydrochloride per unit dose. These dosage forms may preferably be controlled release dosage forms and are formulated for oral administration.

The present invention in one of its most preferred embodiments relates to a controlled release oral pharmaceutical dosage form for treating urinary retention and the pain caused thereby wherein the pharmaceutical dosage form comprises oxycodone hydrochloride and naloxone hydrochloride in a 2:1 weight ratio with oxycodone hydrochloride being present in an amount of 10 to 40 mg per unit dose and naloxone hydrochloride being present in an amount of 5 to 20 mg per unit dose. Preferably the controlled release dosage forms release oxycodone hydrochloride and naloxone hydrochloride by the following *in vitro* dissolution rates when measured according to the European Pharmacopeia paddle test at 50 rpm in 900 ml 0.1 N HCl pH 1.2 using UV detection at 270 nm:
10 - 30 % by weight of oxycodone hydrochloride at 15 min,
30 - 50 % by weight of oxycodone hydrochloride at 1 h,
40 - 65 % by weight of oxycodone hydrochloride at 2 h,
60 - 85 % by weight of oxycodone hydrochloride at 4 h,
70 - 95 % by weight of oxycodone hydrochloride at 7 h,
≥ 80% by weight of oxycodone hydrochloride at 10 h,
10 - 30 % by weight of naloxone hydrochloride at 15 min,
30 - 50 % by weight of naloxone hydrochloride at 1 h,
45 - 65 % by weight of naloxone hydrochloride at 2 h,
60 - 85 % by weight of naloxone hydrochloride at 4 h,
70 - 95 % by weight of naloxone hydrochloride at 7 h, and
≥ 80% by weight of naloxone hydrochloride at 10 h.

In another embodiment the pharmaceutical dosage forms may display the following in vitro dissolution rates when measured according to the United States Pharmacopoeia Basket Method at pH 1.2 using UV detection at 270 nm:
15 to 30 % by weight of oxycodone hydrochloride at 15 min,
45 to 70% by weight of oxycodone hydrochloride at 2 h,
≥ 80% by weight of oxycodone hydrochloride at 7 h,
≥ 90% by weight of oxycodone hydrochloride at 12 h,
15 to 30 % by weight of naloxone hydrochloride at 15 min,
45 to 70% by weight of naloxone hydrochloride at 2 h,
≥ 80% by weight of naloxone hydrochloride at 7 h,
≥ 90% by weight of naloxone hydrochloride at 12 h.

In a particularly preferred embodiment, the dosage form may comprise 10 mg of oxycodone or a pharmaceutically acceptable salt thereof and 5 mg of naloxone or a pharmaceutically acceptable salt thereof, 20 mg of oxycodone or a pharmaceutically acceptable salt thereof and 10 mg of naloxone or a pharmaceutically acceptable salt thereof or 40 mg of oxycodone or a pharmaceutically acceptable salt thereof and 20 mg of naloxone or a pharmaceutically acceptable salt thereof.

Such oral controlled release pharmaceutical dosage forms in accordance with the invention which comprise oxycodone and naloxone or their pharmaceutically acceptable salts in a 2:1 ratio by weight provide a mean tₘₐₓ for oxycodone at about 1 to about 17 hours, at about 2 to about 15 hours, at about 3 to about 8 hours or at about 4 to about 5 hours after single dose administration to healthy human subjects.

Such dosage forms may also provide a mean AUCt value for oxycodone of about 100 ng•h/mL to about 600 ng•h/mL, about 400 ng•h/mL to about 550 ng•h/mL, or about 450 to about 510 ng•h/mL after single dose administration to healthy human subjects.

In yet another embodiment, such dosage forms provide a mean Cₘₐₓ for oxycodone of about 5 ng/mL to about 50 ng/mL, about 30 ng/mL to about 40 ng/mL or about 35 ng/mL after single dose administration to healthy human subjects.

The Cₘₐₓ value indicates the maximum blood plasma concentration of the active agents, i.e. oxycodone and/or naloxone (or of their salts).

The tₘₐₓ value indicates the time point at which the Cₘₐₓ value is reached. In other words, tₘₐₓ is the time point of the maximum observed plasma concentration.

The AUC (Area Under the Curve) value corresponds to the area of the concentration curve. The AUC value is proportional to the amount of active agents, i.e. oxycodone and naloxone absorbed into the blood circulation in total and is hence a measure for the bioavailability.

The AUCt value is the value for the area under the plasma concentration-time curve from the time of administration to the last measurable concentration. AUCt values are usually calculated using the linear trapezoidal method. Where possible, LambdaZ, which is the terminal phase rate constant, is estimated using those points determined to be in the terminal lock-linear phase. t1/2Z, which is the apparent terminal phase half-life, is commonly determined from the ratio of ln2 to LambdaZ. The areas under the plasma concentration-time curve between the last measured point and infinity may be calculated from the ratio of the final observed plasma concentration (Cₗₐₛₜ) to LambdaZ. This is then added to the AUCt to yield AUCinf, which is the area under the plasma concentration-time curve from the time of administration to infinity.

The term "bioavailability" is defined for purposes of the present invention as the extent to which active agents such as oxycodone and naloxone or their pharmaceutically acceptable salts are absorbed from the unit dosage forms.

The term T_{1/2} is defined for purposes of the present invention as the amount of time necessary for one half of the absorbable dose of opioid agonist, preferably oxycodone, and opioid antagonist, preferably naloxone, to be transferred to plasma. This value may be calculated as a "true" value (which would take into account the effect of elimination processes), rather than an "apparent" absorption half-life.

Parameters describing the blood plasma curve can be obtained in clinical trials, first by once-off administration of the active agent such as oxycodone and naloxone to a number of test persons. The blood plasma values of the individual test persons are then averaged, e.g. a mean AUC, Cₘₐₓ and tₘₐₓ value is obtained. In the context of the present invention, pharmacokinetic parameters such as AUC, Cₘₐₓ and tₘₐₓ refer to mean values. Further, in the context of the present invention, *in vivo* parameters such as values for AUC, Cₘₐₓ, tₘₐₓ, or analgesic efficacy refer to parameters or values obtained after administration at steady state or of a single dose to human patients and/or healthy human subjects.

If pharmacokinetic parameters such as mean tₘₐₓ, cₘₐₓ and AUC are measured for healthy human subjects, they are typically obtained by measuring the development of blood plasma values over time in a test population of approximately 16 to 24 healthy human subjects. Regulatory bodies such as the European Agency for the Evaluation of Medicinal Products (EMEA) or the Food and Drug Administration (FDA) will usually accept data obtained from e.g. 20 or 24 test persons. However, initial trials involving fewer participants may also be acceptable.

The term "healthy" human subject in this context refers to a typical male or female of usually Caucasian origin with average values as regards height, weight and physiological parameters such as blood pressure etc. Healthy human subjects for the purposes of the present invention are selected according to inclusion and exclusion criteria which are based on and in accordance with recommendations of the International Conference for Harmonization of Clinical Trials (ICH). For the purposes of the present invention, healthy subjects may be identified according to the inclusion and exclusion criteria as outlaid in Example 7.

Thus, inclusion criteria comprise e.g. an age between ≥18 and ≤45 years; a BMI within the range 19 - 29 kg/m², and within the weight range 60 - 100 kg for males and 55 - 90 kg for females; that females must be non-nursing, non-pregnant, and provide a negative urine ß-hCG pregnancy test within 24 hours before receiving the study medication; generally good health, evidenced by a lack of significantly abnormal findings on medical history, physical examination, clinical laboratory tests, vital signs, and ECG etc.

Exclusion criteria comprise e.g. exposure to any investigational drug or placebo within 3 months of the first dose of study medication, any significant illness within the 30 days before the first dose of study medication, any clinically significant abnormalities identified at prestudy screening for medical history, physical examination or laboratory analyses, use of any prescription medication (except HRT for postmenopausal females and contraceptive medication) in the 21 days, or over the counter medication including acid controllers, vitamins, herbal products and/or mineral supplements in the 7 days, before first dose of study medication, concurrent medical condition known to interfere with gastrointestinal drug absorption (e.g. delayed gastric emptying, mal absorption syndromes), distribution (e.g. obesity), metabolism or excretion (e.g. hepatitis, glomerulonephritis), history of or concurrent medical condition, which in the opinion of the investigator would compromise the ability of the subject to safely complete the study, history of seizure disorders for which subjects required pharmacologic treatment, current history of smoking more than 5 cigarettes a day, subjects with evidence of active or past history of substance or alcohol abuse according to DSM-IV criteria, subjects who reported regular consumption of 2 or more alcoholic drinks per day or have blood alcohol levels of ≥0.5% at screening, donation of more than 500 mL of blood or blood products or other major blood loss in the 3 months before first dose of study medication, any positive results in the prestudy screen for ethanol, opiates, barbiturates, amphetamines, cocaine metabolites, methadone, propoxyphene, phencyclidine, benzodiazepines, and cannabinoids in the specimen of urine collected at screening, known sensitivity to oxycodone, naloxone, or related compounds etc.

If pharmacokinetic parameters such as mean tₘₐₓ, cₘₐₓ and AUC are obtained in patients, the patient group will comprise typically between 10 to 200 patients. A reasonable number of patients will e.g. be 10, 20, 30, 40, 50, 75, 100, 125 or 150 patients. Patients will be selected according to symptoms of the condition to be treated. For the purposes of the present invention, patients may be selected according to the inclusion and exclusion criteria of Example 7. Thus patients will be e.g. ≥ 18 years, suffer from severe chronic pain of tumor and non-tumor origin, will show insufficient efficacy and/or tolerability with a WHO step II or III analgesic etc. A patient will not be considered for determination of pharmacokinetic parameters if there are indications of current alcohol or drug abuse, of current severe cardiovascular and respiratory diseases, of sever liver and renal insufficiency etc.

It is to be understood that values of pharmacokinetic parameters as indicated above and below have been deduced on the basis of the data which were obtained in Example 7, all of which relate to single dose studies in healthy human subjects. However, it is assumed that comparable results will be obtained upon steady state administration in healthy human subject or single dose and steady state administration in human patients.

Pharmacokinetic parameter calculations may be performed with WinNonlin Enterprise Edition, Version 4.1.

The term "steady state" means that a plasma level for a given drug has been achieved and which is maintained with subsequent doses of the drug at a level which is at or above the minimum effective therapeutic level and is below the minimum toxic plasma level for oxycodone. For opioid analgesics such as oxycodone, the minimum effective therapeutic level will be partially determined by the amount of pain relief achieved in a given patient. It will be well understood by those skilled in the medical art that pain measurement is highly subjective and great individual variations may occur among patients. It is clear that after the administration of each dose the concentration passes through a maximum and then again drops to a minimum.

The steady state may be described as follows: At the time t = 0, the time the first dose is administered, the concentration C is also 0. The concentration then passes through a first maximum and then drops to a first minimum. Before the concentration drops to 0, another dose is administered, so that the second increase in concentration doesn't start at 0. Building on this first concentration minimum, the curve passes through a second maximum after the second dose has been administered, which is above the first maximum, and drops to a second minimum, which is above the first minimum. Thus, the blood plasma curve escalates due to the repeated doses and the associated step-by-step accumulation of active agent, until it levels off to a point where absorption and elimination are in balance. This state at which absorption and elimination are in equilibrium and the concentration oscillates constantly between a defined minimum and a defined maximum, is called steady state.

The terms "maintenance therapy" and "chronic therapy" are defined for purposes of the present invention as the drug therapy administered to a patient after a patient is titrated with an opioid analgesic to a steady state as define above.

### EXAMPLES

### Example 1: Production of tablets with different oxycodone/naloxone amounts in a non-swellable diffusion matrix by spray granulation

The following amounts of the listed components were used for the production of oxycodone/naloxone tablets according to the invention.

**Table 1**

| Preparation (designation) | OXN_1 | OXN_2 | OXN-3 |
|---|---|---|---|
| Oxycodone HCl | 20.0 mg | 20.0 mg | 20.0 mg |
| Naloxone HCl | - | 5.0 mg | 10.0 mg |
| Lactose Flow Lac 100 | 59.25 mg | 54.25 mg | 49.25 mg |
| Povidone 30 | 5.0 mg | 5.0 mg | 5.0 mg |
| Surelease® | 10.0 mg solid material | 10.0 mg solid material | 10.0 mg solid material |
| Stearyl alcohol | 25.0 mg | 25.0 mg | 25.0 mg |
| Talcum | 2.5 mg | 2.5 mg | 2.5 mg |
| Mg-Stearate | 1.25 mg | 1.25 mg | 1.25 mg |

The Surelease® E-7-7050 polymer mixture used had the following composition.

**Table 2**

| Surelease® |
|---|
| Ethylcellulose 20 cps |
| Dibutylsebacate |
| Ammoniumhydroxide |
| Oleic acid |
| Siliciumdioxide |
| Water |

For the production of tablets oxycodone HCl, naloxone HCl, Povidone 30 and Lactose Flow Lac 100 were mixed in a tumbling mixer (Bohle) and subsequently spray-granulated with Surelease® E-7-7050 in a fluidized bath granulating device (GPCG3). The material was sieved over a Comill 1.4 mm sieve. An additional granulation step was carried out with melted fatty alcohol in a high-shear mixer (Collette). All tablet cores produced by this approach had a weight of 123 mg, based on dry substance.

### Example 2: Production of tablets with oxycodone and naloxone in a non-swellable diffusion matrix by extrusion

The following amounts of the listed components were used for the production of the oxycodone/naloxone tablets according to the invention.

**Table 3**

| Preparation (designation) | OXN_4 |
|---|---|
| Oxycodone HCl | 20 mg |
| naloxone HCl | 10 mg |
| Kollidon 30 | 6 mg |
| Lactose Flow Lac 100 | 49.25 mg |
| Ethylcellulose 45 cpi | 10 mg |
| Stearyl alcohol | 24 mg |
| Talcum | 2.5 mg |
| Mg-Stearate | 1,25 mg |

The listed amounts of oxycodone HCl, naloxone HCl, ethylcellulose 45 cpi, Povidone 30, stearyl alcohol and Lactose Flow Lac 100 were mixed in a tumbling mixer (Bohle). This mixture was subsequently extruded with a counter-rotating twin screw extruder of the type Micro 18 GGL (Leistritz AG, Nürnberg, Germany). The temperature of heating zone 1 was 25°C, of heating zone 2, 50°C, of heating zones 3 to 5, 60°C, of heating zones 6 to 8, 55°C, of heating zone 9, 60°C and of heating zone 10, 65°C. The screw rotating speed was 150 revolutions per minute (rpm), the resulting melt temperature was 87°C, the feed rate was 1.5 kg/h and the diameter of the nozzle opening was 3 mm. The extruded material was sieved with a Frewitt 0.68 x 1.00 mm sieve. The grinded extrudate was then mixed with talcum and magnesium stearate that had been added over a 1 mm hand sieve and was subsequently pressed into tablets.

In comparison to the oxycodone/naloxone tablets which also have the Surelease®-based non-swellable diffusion matrix produced by spray granulation (see Example 1), extruded preparations comprise less components.

### Example 3: Release profile of the oxycodone/naloxone tablets from Example 1

The release of the active compounds was measured over a time period of 12 hours, applying the Basket Method according to USP at pH 1.2 using HPLC. Tablets OXN_1, OXN_2 and OXN_3 were tested.

The release rates of different oxycodone amounts, independent of the naloxone amount, remain equal (invariant). Correspondingly, invariant release profiles are observed for naloxone at different oxycodone amounts.

**Table 4**

| Time (min) | OXN_1 | OXN_2 | OXN_2 | OXN_3 | OXN_3 |
|---|---|---|---|---|---|
| | Oxyc. | Oxyc. | Nal. | Oxyc. | Nal. |
| 0 | 0 | 0 | 0 | 0 | 0 |
| 15 | 26.1 | 24.9 | 23.5 | 22.8 | 24.1 |
| 120 | 62.1 | 63 | 61 | 57.5 | 60.2 |
| 420 | 91.7 | 94.5 | 91.9 | 89.4 | 93.5 |
| 720 | 98.1 | 99.6 | 96.6 | 95.7 | 100.6 |

The release values refer to oxycodone or naloxone (line 2) and are given as percentages. Oxyc. and Nal. stand for oxycodone and naloxone and indicate the compound measured.

### Example 4: Release profile of oxycodone/naloxone tablets from Example 2 at different pH-values

The release of active compounds from the tablets was measured over a time period of 12 hours at pH 1.2 or for 1 hour at 1.2 and subsequently for 11 hours at pH 6.5. Release rates were determined by the basket method according to USP using HPLC.

The following release rates were measured for 12 hours at pH 1.2:

**Table 5**

| Time (min) | OXN_4 | OXN_4 |
|---|---|---|
| | Oxyc. | Nal. |
| 0 | 0 | 0 |
| 15 | 24.1 | 24.0 |
| 120 | 62.9 | 63.5 |
| 420 | 92.9 | 93.9 |
| 720 | 96.9 | 98.1 |

The following release rates were measured for 1 hour at pH 1.2 and 11 hours at pH 6.5:

**Table 6**

| Time (min) | OXN_4 | OXN_4 |
|---|---|---|
| | Oxyc. | Nal. |
| 0 | 0 | 0 |
| 60 | 48.1 | 49.2 |
| 120 | 65.0 | 64.7 |
| 240 | 83.3 | 81.8 |
| 420 | 94.1 | 92.3 |

The release rates refer to oxycodone and naloxone (line 2) and are given as percentages. Oxyc. and Nal. stand for oxycodone and naloxone and indicate the compound measured.

### Example 5: Production of tablets with different oxycodone/naloxone amounts in a non-swellable diffusion matrix by extrusion

The following amounts of the listed components were used for the production of oxycodone/naloxone tablets according to the invention.

**Table 7**

| Preparation (designation) | OXN_5 | OXN_6 | OXN_7 | OXN_8 |
|---|---|---|---|---|
| Oxycodone HCl | 20 mg | 20 mg | 20 mg | 20 mg |
| Naloxone HCl | 1 mg | 1 mg | 1 mg | 10 mg |
| Lactose Flow Lac 100 | 58.25 mg | 58.25 mg | 58.25 mg | 49.25 mg |
| Kollidon® 30 | 6 mg | 6 mg | 6 mg | 6 mg |
| Ethylcellulose | 10 mg | 10 mg | 10 mg | 10 mg |
| Stearly alcohol | 24 mg | 24 mg | 24 mg | 24 mg |
| Talcum | 1.25 mg | 1.25 mg | 1.25 mg | 1.25 mg |
| Mg-Stearate | 2.5 mg | 2.5 mg | 2.5 mg | 2.5 mg |

Extrusion was performed as described above (Example 2) with the following parameters:

| | | |
|---|---|---|
| OXN_5: | temperature: | 55-63 °C |
| | rpm (screw): | 150 rpm |
| | feeding rate: | 1.5 kg/h |
| | | |
| OXN_6: | temperature: | 55-63 °C |
| | rpm (screw): | 155 rpm |
| | feeding rate: | 1.5 kg/h |
| OXN_7: | temperature: | 55-63 °C |
| | rpm (screw): | 155 rpm |
| | feeding rate: | 1.5 kg/h |
| | | |
| OXN_8: | temperature: | 55-63 °C |
| | rpm (screw): | 160 rpm |
| | feeding rate: | 1.75 kg/h |

Tablet production was performed with a common tabletting device with the following parameters:

| | | |
|---|---|---|
| OXN_5: | rpm: | 40 rpm |
| | Pressure power: | 9 kN |
| | | |
| OXN_6: | rpm: | 42 rpm |
| | Pressure power: | 8.9 kN |
| | | |
| | | |
| OXN_7: | rpm: | 36 rpm |
| | Pressure power: | 9 kN |
| | | |
| | | |
| OXN_8: | rpm: | 36 rpm |
| | Pressure power: | 7.5 kN |

The release of the active compounds was measured over a time period of 12 hours, applying the Basket Method according to USP at pH 1.2 using HPLC. Tablets OXN_5, OXN_6, OXN_7 and OXN_8 were tested.

**Table 8**

| Time (min) | OXN_5 | | OXN_6 | | OXN_7 | | OXN_8 | |
|---|---|---|---|---|---|---|---|---|
| | Oxyc. | Nal. | Oxyc. | Nal. | Oxyc. | Nal. | Oxyc. | Nal. |
| 0 | 0 | 0 | 0 | 0 | 0 | 0 | 0 | 0 |
| 15 | 21.2 | 25.8 | 21.7 | 21.1 | 19.7 | 19.3 | 23.3 | 24.3 |
| 120 | 56.6 | 53.8 | 58.8 | 57.3 | 57.7 | 56.2 | 64.5 | 66.9 |
| 420 | 87.2 | 84.5 | 94.2 | 92.6 | 93.7 | 91.5 | 92.7 | 96.3 |
| 720 | 99.7 | 96.8 | 100.1 | 98 | 100.6 | 97.5 | 93.6 | 97.4 |

The release values refer to oxycodone or naloxone (line 2) and are given as percentages. Oxyc. and Nal. stand for oxycodone and naloxone and indicate the active compound which has been measured.

### Example 6: Production of tablets with oxycodone/naloxone in a non-swellable diffusion matrix by extrusion

In the following example it is set out that using formulations according to the present invention, preparations comprising oxycodone and naloxone with particular release behaviours may be obtained.

The following amounts of the listed components were used for the production of oxycodone/naloxone tablets according to the invention.

**Table 9**

| Preparation (designation) | OXN_9 | OXN_10 | OXN_11 | OXN_12 | OXN_13 | OXN_14 |
|---|---|---|---|---|---|---|
| Oxycodone HCl | 20 mg | 20 mg | 20 mg | 20 mg | 20 mg | 20 mg |
| naloxone HCl | 1 mg | 1 mg | 10 mg | 10 mg | 10 mg | 10 mg |
| Lactose Flow Lac 100 | 56.25mg | 56.25 mg | 54.25 mg | 65.25 mg | 60.25 mg | 55.25mg |
| Kollidon® 30 | 7 mg | 6 mg | 6 mg | 7.25 mg | 7.25 mg | 7.25 mg |
| Ethylcellulose | 11 mg | 12 mg | 10 mg | 12 mg | 12 mg | 12 mg |
| Stearyl alcohol | 24 mg | 24 mg | 24 mg | 28.75 mg | 28.75 mg | 28.75 mg |
| Talcum | 1.25 mg | 1.25 mg | 1.25 mg | 1.25 mg | 1.25 mg | 1.25 mg |
| Mg-Stearate | 2.5 mg | 2.5 mg | 2.5 mg | 2.5 mg | 2.5 mg | 2.5 mg |

Extrusion was performed as described above with the following parameters:

| | | |
|---|---|---|
| OXN_9: | temperature: | 55-63 °C |
| | rpm (screw): | 150 rpm |
| | feeding rate: | 1.5 kg/h |
| | | |
| OXN_10: | temperature: | 55-63 °C |
| | rpm (screw): | 150 rpm |
| | feeding rate: | 1.5 kg/h |
| | | |
| OXN_11: | temperature: | 55-63 °C |
| | rpm (screw): | 160 rpm |
| | feeding rate: | 1.75 kg/h |
| | | |
| OXN_12: | temperature: | 55-63 °C |
| | rpm (screw): | 160 rpm |
| | feeding rate: | 1.75 kg/h |
| OXN_13: | temperature: | 55-63 °C |
| | rpm (screw): | 150 rpm |
| | feeding rate: | 1.5 kg/h |
| | | |
| OXN_14: | temperature: | 55-63 °C |
| | rpm (screw): | 150 rpm |
| | feeding rate: | 1.5 kg/h |

Tablet production was performed with a common tabletting device with the following parameters:

| | | |
|---|---|---|
| OXN_9: | rpm: | 39 rpm |
| | Pressure power: | 11 kN |
| | | |
| OXN_10: | rpm: | 39 rpm |
| | Pressure power: | 10.5 kN |
| | | |
| OXN_11: | rpm: | 36 rpm |
| | Pressure power: | 9.5 kN |
| | | |
| OXN_12: | rpm: | 36 rpm |
| | Pressure power: | 7.8 kN |
| | | |
| OXN_13: | rpm: | 39 rpm |
| | Pressure power: | 9 kN |
| | | |
| OXN_14: | rpm: | 39 rpm |
| | Pressure power: | 7.5 kN |

The release of the active compounds was measured over a time period of 12 hours, applying the Basket Method according to USP at pH 1.2 using HPLC. Tablets OXN_9, OXN_10, OXN_11, OXN_12, OXN_13 and OXN_14 were tested.

**Table 10**

| Time (min) | OXN_9 | | OXN_10 | | OXN_11 | | OXN_12 | | OXN_13 | | OXN_14 | |
|---|---|---|---|---|---|---|---|---|---|---|---|---|
| | Oxyc. | Nal | Oxyc. | Nal | Oxyc. | Nal | Oxyc. | Nal | Oxyc. | Nal | Oxyc. | Nal |
| 0 | 0 | 0 | 0 | 0 | 0 | 0 | 0 | 0 | 0 | 0 | 0 | 0 |
| 15 | 16.6 | 16.2 | 17.4 | 17.2 | 26.1 | 26.8 | 21.8 | 21.9 | 18.5 | 18.2 | 18.4 | 18.2 |
| 120 | 47.6 | 46.9 | 49.6 | 49.7 | 71.1 | 73.0 | 61.2 | 61.8 | 52.8 | 52.8 | 53.3 | 53.3 |
| 420 | 82.7 | 84.5 | 84.6 | 85.7 | 94.3 | 96.6 | 93.2 | 94.7 | 86.3 | 86.3 | 87.2 | 88.2 |
| 720 | 95 | 97 | 95.2 | 95.8 | 94.9 | 97.9 | 96.4 | 97.9 | 94.8 | 94.8 | 95.7 | 96.5 |

The release values refer to oxycodone or naloxone (line 2) and are given as percentages. Oxyc. and Nal. stand for oxycodone and naloxone and indicate the active compound which has been measured.

### Example 7: Pharmacokinetic and bioavailability characteristics of different strengths of a fixed combination of oxycodone and naloxone and a combination of Oxygesic® plus Naloxone CR

### 1. Objective

The objectives of this study were to (i) evaluate the pharmacokinetic and bioavailability parameters of oxycodone and naloxone and their main metabolites when administered as a controlled-release fixed combination tablet formulation; (ii) assess the interchangeability between the 3 different strengths of the fixed combination, OXN 10/5, OXN 20/10 and OXN 40/20; and (iii) compare the pharmacokinetics and bioavailability of the fixed combination formulation with marketed Oxygesic® given together with Naloxone CR tablets;

### 2. Test population

A total of 28 healthy adult, male and female subjects were randomized to receive the study drugs with the aim that 24 subjects would complete the study and provide valid pharmacokinetic data.

### Inclusion Criteria

Subjects who were included in the study were those who met all of the following criteria:
- Males or females of any ethnic group;
- Aged between ≥18 and ≤45 years;
- BMI within the range 19 - 29 kg/m², and within the weight range 60 - 100 kg for males and 55 - 90 kg for females;
- Females must be non-nursing, non-pregnant, and provide a negative urine ß-hCG pregnancy test within 24 hours before receiving the study medication. Female subjects of childbearing potential must be using a reliable form of contraception (e.g. intrauterine device, oral contraceptive, barrier method). Female subjects who were postmenopausal must have been postmenopausal for ≥1 year and, in the absence of HRT, have elevated serum FSH;
- Generally good health, evidenced by a lack of significantly abnormal findings on medical history, physical examination, clinical laboratory tests, vital signs, and ECG. Vital signs (after 3 minutes resting in a supine position) must be within the following ranges: oral body temperature between 35.0 - 37.5 °C; systolic blood pressure, 90 - 140 mmHg; diastolic blood pressure, 50 - 90 mmHg; and pulse rate, 40 - 100 bpm. Blood pressure and pulse were taken again after 3 minutes in a standing position. After 3 minutes standing from a supine position, there should be no more than a 20 mmHg drop in systolic blood pressure, 10 mmHg drop in diastolic blood pressure, and no greater than 20 bpm increase in pulse rate; Written informed consent obtained; Willing to eat all the food supplied during the study.

### Exclusion Criteria

Subjects who were excluded from the study were those who met any of the following criteria:
- Exposure to any investigational drug or placebo within 3 months of their first dose of study medication;
- Any significant illness within the 30 days before their first dose of study medication;
- Any clinically significant abnormalities identified at prestudy screening for medical history, physical examination or laboratory analyses;
- Use of any prescription medication (except HRT for postmenopausal females and contraceptive medication) in the 21 days, or over the counter medication including acid controllers, vitamins, herbal products and/or mineral supplements in the 7 days, before their first dose of study medication;
- Concurrent medical condition known to interfere with gastrointestinal drug absorption (e.g. delayed gastric emptying, mal absorption syndromes), distribution (e.g. obesity), metabolism or excretion (e.g. hepatitis, glomerulonephritis);
- History of, or concurrent medical condition, which in the opinion of the Investigator would compromise the ability of the subject to safely complete the study;
- History of seizure disorders for which subjects required pharmacologic treatment;
- Current history of smoking more than 5 cigarettes a day;
- Subjects with evidence of active or past history of substance or alcohol abuse, according to DSM-IV criteria3, or subjects who, In the investigator's opinion, have demonstrated addictive or substance abuse behaviors;
- Subjects who reported regular consumption of 2 or more alcoholic drinks per day or have blood alcohol levels of ≥0.5% at screening;
- Donation of more than 500 mL of blood or blood products or other major blood loss in the 3 months before their first dose of study medication;
- At risk of transmitting infection via blood samples such as producing a positive HIV test at screening or having participated in a high risk activity for contracting HIV; producing a positive Hepatitis B surface antigen test at screening; producing a positive Hepatitis C antibody test at screening;
- Any positive results in the prestudy screen for ethanol, opiates, barbiturates, amphetamines, cocaine metabolites, methadone, propoxyphene, phencyclidine, benzodiazepines, and cannabinoids in the specimen of urine collected at screening;
- Known sensitivity to oxycodone, naloxone, or related compounds;
- Contraindications and precautions as detailed in the datasheet for Oxygesic@;
- Refusal to allow their primary care physician (if applicable) to be informed;
- The Investigator believed the subject to be unsuitable for a reason not specifically stated in the exclusion criteria.

The demographic data are shown in Table 11.

**Table 11: Subject Demographics and Other Baseline Characteristics: Safety Population**

| | Male (N = 22) | Female (N = 6) | Overall (N = 28) |
|---|---|---|---|
| Characteristics | | | |
| Race, n (%) | | | |
| Caucasian | 22 (100%) | 6 (100%) | 28 (100%) |
| Age (y) | | | |
| Mean ± SD | 32.6 ± 5.28 | 31.0 ± 6.32 | 32.3 ± 5.44 |
| Range (min, max) | 25,41 | 24,42 | 24,42 |
| Height (cm) | | | |
| Mean ± SD | 179.1 ± 4.84 | 168.0 ± 8.72 | 176.7 ± |
| 7.33 | | | |
| Range (min, max) | 165,187 | 159,181 | 159,187 |
| Weight (kg) | | | |
| Mean ± SD | 77.8 ± 9.04 | 67.0 ± 3.03 | 75.5 ± 9.25 |
| Range (min, max) | 62,97 | 63,71 | 62,97 |
| Body Mass Index (kg/m²) | | | |
| Mean ± SD | 24.2 ± 2.56 | 23.9 ± 2.50 | 24.2 ± 2.50 |
| Range (min, max) | 20,29 | 20,27 | 20,29 |

### 3. Study Design, Test Treatment Dose and Mode of Administration

### Preparation of tested products.

A melt extrusion oxycodone/naloxone controlled-release tablet formulation with an oxycodone:naloxone ratio of 2:1 was produced. There are three dose strengths available, namely OXN 10/5, OXN 20/10, and OXN 40/20, where the first number is the mg amount of oxycodone hydrochloride and the second number is the mg amount of naloxone hydrochloride (see Table 12). OXN 20/10 and OXN 40/20 are from the same granulate, while OXN 10/5 has a slightly different formula in regard to the ratio of active ingredients to excipients.

Oxycodone/naloxone tablets (OXN Tablets) according to this example contain a fixed combination of oxycodone and naloxone in the ratio of 2:1. Tablet formulations are summarized below (see Table 12).

The 20/10 mg and 40/20 mg tablets are manufactured from the same granulation with these two tablet strengths being compositionally proportional. Oxycodone/Naloxone prolonged release tablets (OXN) tablets according to this example are controlled release tablets using a matrix of stearyl alcohol and ethylcellulose as the retardant. The tablets contain the combination of oxycodone hydrochloride and naloxone hydrochloride in the strengths 10/5 mg, 20/10 mg and 40/20 mg (both as the hydrochloride). The complete statement of the components and quantitative composition of Oxycodone/Naloxone prolonged release tablets is given below in Table 12.

**Table 12: Oxycodone/Naloxone prolonged release tablets.**

| **Component** | **Quantity (mg/tablet)** | | | **Function** | **Reference to Standard** |
|---|---|---|---|---|---|
| | OXN 10/5 | OXN 20/10 | OXN 40/20 | | |
| Oxycodone hydrochloride ¹⁾ | 10.50 | 21.00 | 42.00 | Active | USP*/ H.S.E. |
| *corresponding to* | | | | | |
| *Oxycodone hydrochloride anhydrous* | *10.00* | *20.00* | *40.00* | | |
| *Oxycodone base* | *9.00* | *18.00* | *36.00* | | |
| Naloxone hydrochloride Dihydrate | 5.45 | 10.90 | 21.80 | Active | Ph. Eur.* |
| *corresponding to* | | | | | |
| *Naloxone hydrochloride anhydrous* | *5.00* | *10.00* | *20.00* | | |
| *Naloxone base* | *4.50* | *9.00* | *18.00* | | |
| Povidone K30 | 5.00 | 7.25 | 14.50 | Binder | Ph. Eur.* |
| Ethylcellulose N 45 | 10.00 | 12.00 | 24.00 | Retardant | Ph. Eur.* |
| Stearyl alcohol | 25.00 | 29.50 | 59.00 | Retardant | Ph. Eur.* |
| Lactose monohydrate | 64.25 | 54.50 | 109.00 | Diluent | Ph. Eur.* |
| Purified talc | 2.50 | 2.50 | 5.00 | Glidant | Ph. Eur.* |
| Magnesium stearate | 1.25 | 1.25 | 2.50 | Lubricant | Ph. Eur.* |
| **Total core** | **123.95** | **138.90** | **277.80** | | |
| **Film Coat °** | | | | | |
| Opadry II HP white - 85F18422 | 3.72 | | | Coating | supplier specificatio n |
| Opadry II HP pink - 85F24151 | | 4.17 | | Coating | supplier specificatio n |
| Opadry II HP yellow 85F32109 | | | 8.33 | Coating | supplier specificatio n |
| Purified talc | 0.12 | 0.14 | 0.28 | Gloss | Ph. Eur.* |
| **Total filmtablet** | **127,79** | **143.21** | **286.41** | | * current Edition |

| | | | | | |
|---|---|---|---|---|---|
| ¹⁾ calculated based on expected moisture content ° qualitative composition : see Table 12 | | | | | |

**Table 13: Qualitative composition of the film coat**

| **Opadry II HP** | **white 85F18422** | **pink 85F24151** | **yellow 85F32109** | **Reference to Standard** |
|---|---|---|---|---|
| Polyvinylalcohol part. hydrolized | + | + | + | Ph. Eur. * |
| Titanium dioxide (E 171) | + | + | + | Ph. Eur. * |
| Macrogol 3350 | + | + | + | Ph. Eur. * |
| Talcum | + | + | + | Ph. Eur. * |
| Iron oxide red (E 172) | | + | | NF* /EC Directive |
| Iron oxide yellow (E 172) | | | + | NF* /EC Directive |
| | | | | * current Edition |

### Study Design

The study was an open-label, single dose, 4-treatment, 4-period, randomized across over study and healthy subjects. The treatments were given orally in the fasted state as follows:
- Treatment A: 4 x tablets of Oxn 10/5
- Treatment B: 2 x tablets of Oxn 20/10
- Treatment C: 1 x tablets of Oxn 40/20

The reference treatment was an Oxygesic® 20 mg tablet. Naloxone was used in the form of Naloxone 10 mg CR spray granulation tablet. Reference treatment was thus
- Treatment D: 2 tablets of Oxygesic® 20 mg and two tablets of Naloxone CR 10 mg.

Duration of treatment included 21 days screening period and four study periods each with a single dose of study drug followed by a seven-day wash-out period. There were post study medical 7 to 10 days after dosing of study period 4 and there were 7 to 10 days after discontinuation from the study. The total duration was 49 to 52 days.

The treatment schedule was a single dose of study drug in each of the four study periods. Each dose of study drug was separated by a seven-day wash-out period.

The enrolled population was defined as the subject population that provided the written informed consent to anticipate in the study. The full analysis population for pharmacokinetics was defined as those subjects, who had at least one valid pharmacokinetic parameter calculated on at least one treatment.

### 4. Pharmacokinetic Assessments

### Drug Concentration Measurements

Blood samples for determining oxycodone, noroxycodone, oxymorphone, noroxymorphone, naloxone, 6β-naloxol and naloxone-3-glucuronide concentrations were obtained for each subject during each of the 4 study periods immediately before dosing; and at 0.5, 1, 1.5, 2, 2.5, 3, 3.5, 4, 5, 6, 8, 10, 12, 16, 24, 28, 32, 36, 48, 72 and 96 hours (22 blood samples per study period) after dosing. Blood was also drawn where possible at the first report of a serious or severe unexpected adverse event and at its resolution.

At each time of plasma determination, 6 mL venous blood was drawn from a forearm vein into a tube containing K2 EDTA anticoagulant. All samples were processed according to common sample handling procedures.

### Pharmacokinetics Parameters

The following pharmacokinetic parameters were calculated from the plasma concentrations of oxycodone, noroxycodone, oxymorphone, noroxymorphone, naloxone, 6β-naloxol and naloxone-3-glucuronide:
- Area under the plasma concentration time curve calculated to the last measurable concentration (AUCt);
- Area under the plasma concentration-time curve, from the time of administration to infinity (AUCINF);
- Maximum observed plasma concentration (Cₘₐₓ);
- Time point of maximum observed plasma concentration (tₘₐₓ);
- Terminal phase rate constant (LambdaZ);
- Apparent terminal phase half-life (t½Z).

For oxycodone, noroxycodone, oxymorphone, noroxymorphone, and naloxone-3-glucuronide, AUC values were given in ng·h/mL, and Cₘₐₓ values in ng/mL. For naloxone and 6β-naloxol, AUC values, due to the low concentrations, were given in pg·h/mL and Cₘₐₓ values in pg/mL.

AUCt, AUCINF and Cₘₐₓ were regarded as the primary parameters.

AUCt were calculated using the linear trapezoidal method. Where possible, LambdaZ was estimated using those points determined to be in the terminal log-linear phase. t½Z was determined from the ratio of ln 2 to LambdaZ. The areas under the plasma concentration-time curve between the last measured point and infinity were calculated from the ratio of the final observed plasma concentration (Cₗₐₛₜ) to LambdaZ. This was then added to the AUCt to yield AUCINF.

All pharmacokinetic calculations were performed with WinNonlin Enterprise Edition, Version 4.1.

### Statistical Methods

Cₘₐₓ and AUCINF of oxycodone were important in order to assess the equivalence of the 4 treatments. AUCt was calculated using the linear trapezoidal method. Where possible, LambdaZ was estimated using those points determined to be in the terminal log-linear phase. t½Z were determined from the ratio of ln 2 to LambdaZ. The areas under the plasma concentration-time curve between the last measured point and infinity were calculated from the ratio of the final observed plasma concentration (Cₗₐₛₜ) to LambdaZ. This was added to the AUCt to yield the area under the plasma concentration-time curve between the time of administration and infinity (AUCINF).

The dose adjusted relative systemic availabilities (Frelt, and FrelINF) and the Cₘₐₓ ratio were obtained from the ratio of AUCt, AUCINF and Cₘₐₓ values, respectively, for differences defined in the following comparisons of interest:
fixed combination A vs. open combination D
fixed combination B vs. open combination D
fixed combination C vs. open combination D
fixed combination A vs. fixed combination B
fixed combination A vs. fixed combination C
fixed combination B vs. fixed combination C

The full analysis population for pharmacokinetics were used for these analyses.

The metabolite: parent drug AUCt and AUCINF ratios were estimated for each treatment, where possible.

### 5. Clinical Pharmacology Results

Mean observed plasma concentration - time curves for oxycodone, naloxone-3-glucuronide, naloxone, noroxycodone, oxymorphone, noroxymorphone and 6-β-naloxol are presented in Figures 1 to 7.

Pharmacokinetic parameters for oxycodone, naloxone-3-glucuronide and naloxone are presented in Tables 14 to 19 respectively.

**Table 14: Summary of Pharmacokinetic Parameters for Oxycodone by Treatment: Full Analysis Population for Pharmacokinetics**

| **Pharmacokinetic parameter** | **4 x OXN 10/5** | **2 x OXN 20/10** | **1 x OXN 40/20** | **2 x Oxygesic 20 + 2 x Naloxone 10** |
|---|---|---|---|---|
| **AUCt (ng.h/mL)** | | | | |
| N | 24 | 23 | 23 | 23 |
| Arithmetic Mean | 473.49 | 491.22 | 488.89 | 502.28 |
| (SD) | (72.160) | (82.181) | (91.040) | (84.128) |
| Geometric Mean | 468.29 | 484.58 | 481.08 | 495.72 |

| **AUCINF (ng.h/mL)** | | | | |
|---|---|---|---|---|
| N | 24 | 22 | 22 | 22 |
| Arithmetic Mean | 475.06 | 497.17 | 491.22 | 509.11 |
| (SD) | (72.182) | (81.687) | (93.458) | (82.963) |
| Geometric Mean | 469.87 | 490.65 | 483.04 | 502.80 |

| **Cmax (ng/mL)** | | | | |
|---|---|---|---|---|
| N | 24 | 23 | 23 | 23 |
| Arithmetic Mean | 34.91 | 35.73 | 34.46 | 40.45 |
| (SD) | (4.361) | (4.931) | (5.025) | (4.706) |
| Geometric Mean | 34.66 | 35.41 | 34.12 | 40.19 |

| **tmax (h)** | | | | |
|---|---|---|---|---|
| N | 24 | 23 | 23 | 23 |
| Median | 3.5 | 4.0 | 3.0 | 2.5 |
| (Min,Max) | (1.0, 6.0) | (2.0, 8.0) | (1.0, 6.0) | (0.5, 8.0) |

| **t1/2Z** | | | | |
|---|---|---|---|---|
| N | 24 | 22 | 22 | 22 |
| Arithmetic Mean | 4.69 | 4.87 | 4.83 | 5.01 |
| (SD) | (0.775) | (0.995) | (0.975) | (0.802) |

**Table 15: Oxycodone Summary of Ratios for AUCt, AUCINF, Cₘₐₓ and Differences for tₘₐₓ and Half-Life - Full Analysis Population for Pharmacokinetics**

| **Pharmacokinetic metric** | **4 x OXN 10/5 / 2x Oxygesic 20+2x Naloxone 10** | **2 x OXN 20/10 / 2 x Oxygesic 20 + 2 x Naloxone 10** | **1 x OXN 40/20 / 2 x Oxygesic 20 + 2 x Naloxone 10** | **4 x OXN 10/5 / 2 x OXN 20/10** | **4 x OXN 10/5 / 1 x OXN 40/20** | **2 x OXN 20/10 / 1 x OXN 40/20** |
|---|---|---|---|---|---|---|
| **AUCt (ng.h/mL)** | | | | | | |
| Ratio (%) | 94.9 | 98.2 | 98.0 | 96.7 | 96.8 | 100.2 |
| 90%CI | 91.5, 98.5 | 94.5, 102.0 | 94.4, 101.7 | 93.1, 100.4 | 93.3, 100.5 | 96.5, 104.0 |

| **AUCINF(ng.h/ mL)** | | | | | | |
|---|---|---|---|---|---|---|
| Ratio (%) | 94.5 | 98.2 | 97.8 | 96.2 | 96.5 | 100.4 |
| 90%CI | 90.9, 98.1 | 94.5, 102.1 | 94.1, 101.7 | 92.6, 99.9 | 92.9, 100.3 | 96.5, 104 .3 |

| **Cmax (ng/mL)** | | | | | | |
|---|---|---|---|---|---|---|
| Ratio (%) | 86.2 | 88.4 | 85.8 | 97.5 | 100.5 | 103.1 |
| 90%CI | 82.2, 90.4 | 84.2, 92.8 | 81.8, 90.0 | 92.9, 102.3 | 95.8, 105.4 | 98.2, 108.1 |

| **tmax (h)** | | | | | | |
|---|---|---|---|---|---|---|
| Difference | 0.49 | 1.11 | 0.14 | -0.63 | 0.35 | 0.97 |
| 90%CI | -0.19, 1.16 | 0.42, 1.80 | -0.54, 0.82 | -1.31, 0.05 | -0.33, 1.02 | 0.29, 1.6 6 |

| **t1/2Z (h)** | | | | | | |
|---|---|---|---|---|---|---|
| Difference | -0.27 | -0.11 | -0.11 | -0.16 | -0.16 | 0.00 |
| 90%CI | -0.60, 0.05 | -0.44, 0.23 | -0.44, 0.22 | -0.49, 0.16 | -0.49, 0.16 | -0.33, 0.33 |

**Table 16: Summary of Pharmacokinetic Parameters for Naloxone-3-glucuronide by Treatment: Full Analysis Population for Pharmacokinetics**

| **Pharmacokinetic parameter** | **4 x OXN 10/5** | **2 x OXN 20/10** | **1 x OXN 40/20** | **2 x Oxygesic 20 + 2 x Naloxone 10** |
|---|---|---|---|---|
| **AUCt (pg.h/mL)** | | | | |
| N | 24 | 23 | 23 | 23 |
| Arithmetic Mean | 539.93 | 522.45 | 520.10 | 523.37 |
| (SD) | (142.241) | (128.569) | (133.175) | (119.752) |
| Geometric Mean | 520.14 | 506.63 | 502.26 | 509.38 |

| **AUCINF(pg.h/mL)** | | | | |
|---|---|---|---|---|
| N | 22 | 21 | 22 | 22 |
| Arithmetic Mean | 562.53 | 520.97 | 527.94 | 537.25 |
| (SD) | (130.732) | (133.172) | (135.424) | 110.829 |
| Geometric Mean | 546.73 | 504.34 | 509.62 | 525.91 |

| **Cmax (pg/mL)** | | | | |
|---|---|---|---|---|
| N | 24 | 23 | 23 | 23 |
| Arithmetic Mean | 62.01 | 63.62 | 61.95 | 63.55 |
| (SD) | (15.961) | (19.511) | (18.369) | (16.748) |
| Geometric Mean | 59.93 | 60.70 | 59.34 | 61.55 |

| **tmax (h)** | | | | |
|---|---|---|---|---|
| N | 24 | 23 | 23 | 23 |
| Median | 1.0 | 0.5 | 1.0 | 1.0 |
| (Min,Max) | (0.5, 3.0) | (0.5, 6.0) | (0.5, 3.0) | (0.5, 6.0) |

| **t1/2Z** | | | | |
|---|---|---|---|---|
| N | 22 | 21 | 22 | 22 |
| Arithmetic Mean | 8.48 | 7.93 | 7.81 | 7.66 |
| (SD) | (3.066) | (2.402) | (2.742) | (1.717) |

**Table 17: Naloxone-3-Glucuronide Summary of Ratios for AUCt, AUCINF, Cₘₐₓ and Differences for tₘₐₓ and Half-Life - Full Analysis Population for Pharmacokinetics**

| **Pharmacokinetic metric** | **4 x OXN 10/5 / 2 x Oxygesic 20 + 2x Naloxone 10** | **2 x OXN 20/10 / 2 x Oxygesic 20 + 2x Naloxone 10** | **1 x OXN 40/20 / 2 x Oxygesic 20 + 2x Naloxone 10** | **4 x OXN 10/5 / 2 x OXN 20/10** | **4 x OXN 10/5 / 1 x OXN 40/20** | **2 x OXN 20/10 / 1 x OXN 40/20** |
|---|---|---|---|---|---|---|
| **AUCt (pg.h/mL)** | | | | | | |
| Ratio (%) | 101.0 | 98.8 | 98.6 | 102.2 | 102.4 | 100.2 |
| 90%CI | 95.6, 106.8 | 93.4, 104.5 | 93.3, 104.3 | 96.7, 108.1 | 97.0, 108.2 | 94.8, 105.9 |
| **AUCINF(pg.h/m L)** | | | | | | |
| Ratio (%) | 102.1 | 98.2 | 99.0 | 104.0 | 103.1 | 99.2 |
| 90%CI | 96.3, 108.3 | 92.3, 104.2 | 93.4, 105.0 | 97.9, 110.5 | 97.3, 109.3 | 93.5, 105.2 |
| **Cmax (pg/mL)** | | | | | | |
| Ratio (%) | 95.4 | 96.5 | 95.1 | 98.8 | 100.3 | 101.5 |
| 90%CI | 88.5, 102.8 | 89.4, 104.1 | 88.2, 102.5 | 91.7, 106.6 | 93.1, 108.0 | 94.1, 109.3 |
| **tmax (h)** | | | | | | |
| Difference | -0.34 | -0.16 | -0.42 | -0.18 | 0.08 | 0.26 |
| 90%CI | -0.84, 0.17 | -0.67, 0.35 | -0.93, 0.10 | -0.69, 0.33 | -0.43, 0.59 | -0.26, 0.77 |
| **t1/2Z (h)** | | | | | | |
| Difference | 0.87 | 0.37 | 0.32 | 0.50 | 0.56 | 0.06 |
| 90%CI | -0.02, 1.77 | -0.53, 1.28 | -0.58, 1.21 | -0.41, 1.41 | -0.33, 1.45 | -0.85, 0.96 |

**Table 18: Summary of Pharmacokinetic Parameters for Naloxone by Treatment: Full Analysis Population for Pharmacokinetics.**

| **Pharmacokinetic parameter** | **4 x OXN 10/5** | **2 x OXN 20/10** | **1 x OXN 40/20** | **2 x Oxygesic 20 + 2 x Naloxone 10** |
|---|---|---|---|---|
| **AUCt (pg.h/mL)** | | | | |
| N | 24 | 23 | 23 | 23 |
| Arithmetic Mean | 0.84 | 0.89 | 0.87 | 0.97 |
| (SD) | (0.656) | (0.749) | (0.718) | (0.976) |
| Geometric Mean | 0.67 | 0.70 | 0.68 | 0.72 |

| **AUCINF(pg.h/m L)** | | | | |
|---|---|---|---|---|
| N | 2 | 6 | 0 | 1 |
| Arithmetic Mean | - | 1.64 | - | - |
| (SD) | - | (1.043) | - | - |
| Geometric Mean | - | 1.45 | - | - |

| **Cmax (pg/mL)** | | | | |
|---|---|---|---|---|
| N | 24 | 23 | 23 | 23 |
| Arithmetic Mean | 0.07 | 0.08 | 0.08 | 0.08 |
| (SD) | (0.065) | (0.106) | (0.071) | (0.101) |
| Geometric Mean | 0.06 | 0.06 | 0.06 | 0.06 |

| **tmax (h)** | | | | |
|---|---|---|---|---|
| N | 24 | 23 | 23 | 23 |
| Median | 4.0 | 5.0 | 2.0 | 1.0 |
| (Min,Max) | (0.5, 12.0) | (0.5, 24.0) | (0.5, 12.0) | (0.5, 24.0) |

| **t1/2Z** | | | | |
|---|---|---|---|---|
| N | 4 | 9 | 4 | 4 |
| Arithmetic Mean | 9.89 | 12.85 | 13.83 | 11.02 |
| (SD) | (3.137) | (11.924) | (1.879) | (1.075) |

**Table 19: Naloxone Summary of Ratios for AUCt, AUCINF, Cₘₐₓ and Differences for Tₘₐₓ and Half-Life - Full Analysis Population for Pharmacokinetics.**

| **Pharmacokinetic metric** | **4 x OXN 10/5 / 2 x Oxygesic 20 + 2 x Naloxone 10** | **2 x OXN 20/10 / 2 x Oxygesic 20 + 2 x Naloxone 10** | **1 x OXN 40/20 / 2 x Oxygesic 20 + 2 x Naloxone 10** | **4 x OXN 10/5 / 2 x OXN 20/10** | **4 x OXN 10/5/1x OXN 40/20** | **2 x OXN 20/10 / 1 x OXN 40/20** |
|---|---|---|---|---|---|---|
| **AUCt (pg.h/mL)** | | | | | | |
| Ratio (%) | 94.2 | 99.4 | 94.1 | 94.7 | 100.1 | 105.7 |
| 90%CI | 82.0, 108.2 | 86.3, 114.5 | 81.8, 108.1 | 82.4, 108.9 | 87.3, 114.9 | 92.0, 121.5 |

| **AUCINF (pg.h/mL)** | | | | | | |
|---|---|---|---|---|---|---|
| Ratio (%) | - | - | - | - | - | - |
| 90%CI | -- | -- | -- | -- | -- | -- |

| **Cmax (pg/mL)** | | | | | | |
|---|---|---|---|---|---|---|
| Ratio (%) | 102.4 | 108.8 | 104.1 | 94.1 | 98.4 | 104.5 |
| 90%CI | 88.0, 119.2 | 93.1, 127.0 | 89.3, 121.2 | 80.8, 109.7 | 84.6, 114.4 | 89.7, 121.8 |

| **tmax (h)** | | | | | | |
|---|---|---|---|---|---|---|
| Difference | -0.71 | 0.12 | -2.03 | -0.83 | 1.32 | 2.15 |
| 90%CI | -2.96, 1.54 | -2.17, 2.42 | -4.31, 0.24 | -3.10, 1.44 | -0.93, 3.57 | -0.12, 4.43 |

| **t1/2Z (h)** | | | | | | |
|---|---|---|---|---|---|---|
| Difference | -3.55 | 0.79 | 2.30 | -4.35 | -5.85 | -1.51 |
| 90%CI | -12.92, 5.82 | -23.09, 24.67 | -22.06, 26.67 | -28.49, 19.80 | -30.48, 18.77 | -8.80, 5.78 |

### 6. Data Analysis

### a) Oxycodone Results

### - AUCt

The AUCt values obtained for oxycodone were very consistent between the treatments. Each of the treatments had a mean AUCt value of between 473 ng.h/mL (4 x OXN 10/5) and 502 ng.h/mL (2 x Oxygesic 20 mg & 2 x naloxone CR 10 mg).

In terms of AUCt, each of the fixed combination tablets provided an equivalent availability of oxycodone to the reference treatment, and to each other. All of the relative bioavailability calculations had 90% confidence intervals that were within the 80 - 125% limits of acceptability for bioequivalence.

### - t1/2Z

The t1/2Z values obtained for oxycodone were consistent between the treatments. Each of the treatments had a mean t1/2Z value of between 4.69 h (4 x OXN 10/5), and 5.01 h (2 x Oxygesic 20 mg & 2 x naloxone CR 10 mg). There were no statistical differences between the t1/2Z values for the treatments for any of the comparisons that were made.

### - AUCINF

The AUCINF values obtained for oxycodone were very consistent between the treatments. Each of the treatments had a mean AUCINF value of between 475 ng.h/mL (4 x OXN 10/5) and 509 ng.h/mL (2 x Oxygesic 20 mg & 2 x naloxone CR 10 mg).

In terms of AUCINF, each of the fixed combination tablets provided an equivalent availability of oxycodone to the reference treatment, and to each other. All of the relative bioavailability calculations had 90% confidence intervals that were within the 80 - 125% limits of acceptability for bioequivalence.

### - Cₘₐₓ

The Cₘₐₓ values obtained for oxycodone were consistent between the fixed combination treatments, and ranged from 34.46 ng/mL (1 x OXN 40/20) to 35.73 ng/mL (2 x OXN 20/10). The mean Cₘₐₓ value for 2 x Oxygesic 20 mg & 2 x naloxone CR 10 mg was slightly higher at 40.45 ng/mL.

The Cₘₐₓ ratios comparing the fixed combination tablets with each other ranged from 97.5% to 103.1%, and each had 90% confidence intervals within 80 - 125%. The higher mean Cₘₐₓ value for 2 x Oxygesic 20 mg & 2 x naloxone CR 10 mg meant that the Cₘₐₓ ratios comparing the fixed combination tablet with the reference product were lower, ranging from 85.8% to 88.4%. However, these Cₘₐₓ ratios were still associated with 90% confidence intervals that were within 80 - 125%.

### - tₘₐₓ

The median tₘₐₓ values for the fixed combination tablets ranged from 3 h (1 x OXN 40/20) to 4 h (2 x OXN 20/10). The difference between these two treatments, although apparently small, was statistically significant. The median tₘₐₓ for 2 x Oxygesic 20 mg & 2 x naloxone CR 10 mg was 2.5 h, and there was a statistically significant difference between this reference treatment and 2 x OXN 20/10.

### b) Naloxone-3-Glucuronide Results

### - AUCt

The AUCt values obtained for naloxone-3-glucuronide were very consistent between the treatments. Each treatment had a mean AUCt value of between 520 ng.h/mL (1 x OXN 40/20) and 540 ng.h/mL (4 x OXN 10/5).

In terms of AUCt, each of the fixed combination tablets provided an equivalent availability of naloxone-3-glucuronide to the reference treatment, and to each other. All of the relative bioavailability calculations had 90% confidence intervals that were within the 80 - 125% limits of acceptability for bioequivalence.

### - t1/2Z

The t1/2Z values obtained for naloxone-3-glucuronide were consistent between the treatments. Each of the treatments had a mean t1/2Z value of between 7.66 h (2 x Oxygesic 20 mg & 2 x naloxone CR 10 mg) and 8.48 h (4 x OXN 10/5). There were no statistical differences between the t1/2Z values for the treatments for any of the comparisons that were made.

### - AUCINF

The AUCINF values obtained for naloxone-3-glucuronide were very consistent between the treatments. Each of the treatments had a mean AUCINF value of between 521 ng.h/mL (2 x OXN 20/10) and 563 ng.h/mL (4 x OXN 10/5).

In terms of AUCINF, each of the fixed combination tablets provided an equivalent availability of naloxone-3-glucuronide to the reference treatment, and to each other. All of the bioavailability calculations had 90% confidence intervals that were within the 80 - 125% limits of acceptability for bioequivalence.

### - Cₘₐₓ

The Cₘₐₓ values obtained for naloxone-3-glucuronide were consistent between the treatments. Each of the treatments had a mean Cₘₐₓ value that range from 61.95 ng.mL (1 x OXN 40/20) to 63.62 ng.mL (2 x OXN 20/10).

Each of the fixed combination tablets provided an equivalent naloxone-3-glucuronide Cₘₐₓ to the reference treatment, and to each other. All of the Cₘₐₓ ratio calculations had 90% confidence intervals that were within the 80 - 125% limits of acceptability for bioequivalence.

### - tₘₐₓ

The median tₘₐₓ values for all the treatments ranged from 0.5 h (2 x OXN 20/10) to 1 h (4 x OXN 10/5, 1 x OXN 40/20 and 2 x Oxygesic 20 mg & 2 x naloxone CR 10 mg). There were no significant differences between the median tₘₐₓ values for any of the treatments.

### - Naloxone-3-glucuronide : naloxone AUCt ratios

The mean naloxone-3-glucuronide : naloxone AUCt ratios ranged from 852.25 (2 x Oxygesic 20 mg & 2 x naloxone CR 10 mg) to 933.46 (4 x OXN 10/5).

### - Naloxone-3-glucuronide : naloxone AUCINF ratios

The lack of AUCINF estimates for naloxone meant that mean naloxone-3-glucuronide : naloxone AUCINF ratios were only able to be calculated for 2 x OXN 20/10 tablets. These provided a mean naloxone-3-glucuronide : naloxone AUCINF ratio of 414.56, based on 5 subjects' data.

### c) Naloxone Results

Naloxone concentrations were low, as was anticipated; therefore, these results did not support a full pharmacokinetic assessment.

### - AUCt

The AUCt values obtained for naloxone were consistent between the treatments. Each of the treatments had a mean AUCt value of between 0.84 ng.h/mL (2 x OXN 20/10) and 0.97 ng.h/mL (2 x Oxygesic 20 mg & 2 x naloxone CR 10 mg).

In terms of AUCt, each of the fixed combination tablets provided an equivalent availability of naloxone to the reference treatment, and to each other. All of the bioavailability calculations had 90% confidence intervals that were within the 80 - 125% limits of acceptability for bioequivalence.

### - t1/2Z

It was not possible to calculate t1/2Z values for naloxone for all of the subjects with confidence, because the plasma concentrations in the terminal part of the profile did not always approximate to a straight line when plotted on a semi-logarithmic scale. The mean values were based on numbers of subjects ranging from 4 to 9.

The mean t1/2Z values obtained for naloxone ranged from between 9.89 h (4 x OXN 10/5) to 13.83 h (1 x OXN 40/20). There were a wide range of t1/2Z values contributing to the means, however, there were no statistical differences between the t1/2Z values for the treatments for any of the comparisons that were made.

### - AUCINF

AUCINF values were calculated for those subjects with an estimable t1/2Z value. Some of the AUCINF values were not reportable because the extrapolated portion of the AUC accounted for more than 20% of the AUCINF value. A mean AUCINF value, of 1.64 ng.h/mL, was reportable for 2 x OXN 20/10 tablets only. None of the other treatments had sufficient data to report a mean AUCINF value. There were insufficient data to make comparisons between the treatments.

### - Cₘₐₓ

Each of the treatments had a mean Cₘₐₓ value of between 0.07 ng/mL (4 x OXN 10/5) and 0.08 ng/mL (2 x OXN 20/10, 1 x OXN 40/20 and 2 x Oxygesic 20 mg & 2 x naloxone CR 10 mg).

Each of the fixed combination tablets provided an equivalent naloxone Cₘₐₓ to each other. All of the Cₘₐₓ ratios comparing the fixed combination tablets had 90% confidence intervals that were within the 80 - 125% limits of acceptability for bioequivalence.

When the fixed combination tablets were compared with the reference product, the 2 x OXN 20/10 tablets versus 2 x Oxygesic 20 mg & 2 x naloxone CR 10 mg had a 90% confidence interval that was above the 80 - 125% limit of acceptability for bioequivalence. The remaining fixed combination tablets provided an equivalent naloxone Cₘₐₓ to the reference product.

### - tₘₐₓ

The median tₘₐₓ values for the treatments ranged from 1 h (2 x Oxygesic 20 mg & 2 x naloxone CR 10 mg) to 5 h (2 x OXN 20/10). There were a wide range of tₘₐₓ values for each of the treatments. There were no significant differences between the median tₘₐₓ values for any of the treatments.

### 7. Clinical Pharmacology Discussion and Conclusions

Low oral bioavailability prevents the complete pharmacokinetic assessment of naloxone. This was confirmed as the low plasma concentrations meant that it was not possible to estimate AUCINF values for naloxone for most of the subjects. Naloxone-3-glucuronide was present in the plasma in much higher concentrations, and AUCINF estimates were obtained for naloxone-3-glucuronide for the majority of subjects. The conclusions for the naloxone component of the fixed combination tablets were based on naloxone-3-glucuronide parameters.

### a) Oxycodone

The mean plasma oxycodone concentration-time curves for 2 x Oxygesic 20 mg & 2 x naloxone CR 10 mg and the fixed combination tablets were almost superimposable.

A bioequivalence assessment was made for oxycodone. Each of the bioequivalence comparisons had 90% confidence intervals that were within the limits of acceptability for bioequivalence for Frelt, FrelINF and Cₘₐₓ ratio. The oxycodone results indicate that each of the fixed combination tablet strengths were bioequivalent, both to each other and also to Oxygesic given together with naloxone CR tablet. There were no statistical differences between any of the tₘₐₓ or t1/2Z values for any of the treatments, further confirming the similarity of the products.

The plasma oxycodone concentrations achieved after administration of the reference product were similar to dose-adjusted oxycodone concentrations seen after administration of OxyContin in a previous study. The mean Cₘₐₓ values for the fixed combination tablets were slightly lower, but when these were compared with the reference product, the Cₘₐₓ ratios had confidence intervals that were within the limits of acceptability for bioequivalence.

### b) Naloxone

The mean plasma naloxone concentrations were low, less than 0.1 ng/mL, and appeared to be biphasic, with a second peak occurring at between 8 to 16 hours.

Even though all of the subjects did have quantifiable plasma naloxone concentrations, individual subjects' plasma naloxone concentrations were low and highly variable. The maximum observed plasma naloxone concentrations were 0.07 to 0.08 ng/mL.

The pharmacokinetic profiles of naloxone from earlier studies were examined. On average, the mean Cₘₐₓ values from these studies, dose-adjusted to a single dose of 1 mg, ranged between 4 and 15 pg/mL confirming that the low plasma naloxone concentrations observed here were consistent with those levels measured in earlier studies.

A bioequivalence assessment was made for naloxone. The variability of the plasma naloxone concentrations did not allow for an estimate of AUCINF, or therefore FrelINF values. The bioavailability estimate was based on Frelt values. Each of the bioavailability comparisons had 90% confidence intervals that were within the limits of acceptability for bioequivalence. The mean Cₘₐₓ values for naloxone were comparable, and five out of the six bioavailability comparisons had 90% confidence intervals that met the criteria for bioequivalence.

The tₘₐₓ and t1/2Z values for the treatments were variable, however there were no significant differences between any of the treatments for these two parameters.

As expected, the levels of naloxone-3-glucuronide seen in the plasma after administration of the fixed combination tablets and Oxygesic plus naloxone, were much higher than the levels of naloxone that were achieved, resulting in naloxone-3-glucuronide : naloxone AUCt ratios of around 900. 6β-naloxol was also measured in higher quantities than naloxone, resulting in 6β-naloxol : naloxone AUCt ratios of around 22. These metabolite : parent AUCt ratios were consistent across the fixed combination tablets and the reference treatment.

### c) Naloxone-3-glucuronide

The mean plasma naloxone-3-glucuronide levels were higher than naloxone, and it was possible to make a bioavailability assessment based on FrelINF values.

A bioequivalence assessment was made for naloxone-3-glucuronide. Each of the bioequivalence comparisons had 90% confidence intervals that were within the limits of acceptability for bioequivalence for Frelt, FrelINF and Cₘₐₓ ratio. The naloxone-3-glucuronide results indicate that each of the fixed combination tablet strengths were bioequivalent to each other, and to Oxygesic plus naloxone. There were no statistical differences between any of the tₘₐₓ or t1/2Z values for any of the treatments, further confirming the similarity of the products.

Such formulations were then tested in clinical studies and pharmacovigilance studies versus a controlled release oxycodone hydrochloride formulation, Oxygesic® for analgesic efficacy. These clinical trials and pharmacovigilance studies involving more than 1000 patients have shown that for treatment with the oxycodone/naloxone combination, typical opioid-induced side effects such as urinary disorders including urinary retention are reduced to rare frequency. This illustrates that naloxone is capable of treating urinary retention.

## Claims

1. Pharmaceutical composition comprising at least one opioid antagonist or a pharmaceutically acceptable salt thereof and at least one opioid agonist or a pharmaceutically acceptable salt thereof for use in treating urinary retention, wherein said opioid antagonist is naloxone or a pharmaceutically acceptable salt thereof and wherein said pharmaceutical composition is formulated for oral application.

2. Pharmaceutical composition for use according to claim 1, wherein said opioid antagonist is naloxone hydrochloride.

3. Pharmaceutical composition for use according to claim 1, wherein said opioid agonist is selected from the group comprising oxycodone, morphine, hydromorphone, oxymorphone and pharmaceutically acceptable salts thereof.

4. Pharmaceutical composition for use according to claim 3, wherein said opioid agonist is oxycodone hydrochloride.

5. Pharmaceutical composition for use according to any of claims 1 to 4, wherein said composition comprises naloxone hydrochloride and oxycodone hydrochloride as the sole pharmaceutically active agents in a 1:2 ratio by weight.

6. Pharmaceutical composition for use according to any of claims 1 to 5, wherein said composition is an immediate release dosage form.

7. Pharmaceutical composition for use according to any of claims 1 to 5, wherein said composition is a controlled release dosage form.

8. Pharmaceutical composition for use according to claim 7, wherein said controlled release dosage form comprises a controlled release matrix or a controlled release coating.

9. Pharmaceutical composition for use according to claim 7 or 8, wherein said dosage form comprises naloxone or a pharmaceutically acceptable salt thereof and wherein said dosage form releases naloxone or a pharmaceutically acceptable salt thereof when measured according to the European Pharmacopoeia paddle test at a rate of:
10 - 30 % by weight of naloxone or said salt thereof at 15 min,
30 - 50 % by weight of naloxone or said salt thereof at 1 h,
45 - 65 % by weight of naloxone or said salt thereof at 2 h,
60 - 85 % by weight of naloxone or said salt thereof at 4 h,
70 - 95 % by weight of naloxone or said salt thereof at 7 h, and
≥ 80% by weight of naloxone or said salt thereof at 10 h.

10. Oral pharmaceutical composition comprising at least oxycodone or a pharmaceutically acceptable salt thereof and naloxone or a pharmaceutically acceptable salt thereof for use in treating pain in patients which otherwise have to discontinue opioid-based pain therapy for development of urinary retention, wherein said composition is a controlled release dosage form.

11. Pharmaceutical composition for use according to claim 10, wherein said composition comprises oxycodone hydrochloride and naloxone hydrochloride.

12. Pharmaceutical composition for use according to claim 10 or 11, wherein said composition comprises oxycodone and naloxone or their hydrochloride salts in a 2:1 ratio by weight.

13. Pharmaceutical composition for use according to any of claims 10 to 12, wherein said composition comprises oxycodone or a pharmaceutically acceptable salt thereof in an amount of 5 to 160 mg per unit dose and naloxone or a pharmaceutically acceptable salt thereof in an amount of 2.5 to 80 mg per unit dose.

14. Pharmaceutical composition for use according to any of claims 10 to 13, wherein said controlled release dosage form comprises a controlled release matrix or a controlled release coating.

15. Pharmaceutical composition for use according to claim 14, wherein said dosage form comprises oxycodone or a pharmaceutically acceptable salt thereof and naloxone or a pharmaceutically acceptable salt thereof and wherein said dosage form releases oxycodone or a pharmaceutically acceptable salt thereof and naloxone oor a pharmaceutically acceptable salt thereof when measured according to the European Pharmacopoeia paddle test at a rate of:
10 - 30 % by weight of oxycodone or said salt thereof at 15 min,
30 - 50 % by weight of oxycodone or said salt thereof at 1 h,
45 - 65 % by weight of oxycodone or said salt thereof at 2 h,
60 - 85 % by weight of oxycodone or said salt thereof at 4 h,
70 - 95 % by weight of oxycodone or said salt thereof at 7 h, and
≥ 80% by weight of oxycodone or said salt thereof at 10 h, and
10 - 30 % by weight of naloxone or said salt thereof at 15 min,
30 - 50 % by weight of naloxone or said salt thereof at 1 h,
45 - 65 % by weight of naloxone or said salt thereof at 2 h,
60 - 85 % by weight of naloxone or said salt thereof at 4 h,
70 - 95 % by weight of naloxone or said salt thereof at 7 h, and
≥ 80% by weight of naloxone or said salt thereof at 10 h.

## Patentansprüche

1. Pharmazeutische Zusammensetzung umfassend mindestens einen Opioid-Antagonisten oder ein pharmazeutisch annehmbares Salz davon und mindestens einen Opioid-Agonisten oder ein pharmazeutisch annehmbares Salz davon zur Verwendung in der Behandlung von Harnverhalt, wobei besagter Opioid-Antagonist Naloxon oder ein pharmazeutisch annehmbares Salz davon ist und wobei besagte pharmazeutische Zusammensetzung zur oralen Anwendung formuliert ist.

2. Pharmazeutische Zusammensetzung zur Verwendung gemäß Anspruch 1, wobei besagter Opioid-Antagonist Naloxonhydrochlorid ist.

3. Pharmazeutische Zusammensetzung zur Verwendung gemäß Anspruch 1, wobei besagter Opioid-Antagonist ausgewählt wird aus der Gruppe umfassend Oxycodon, Morphin, Hydromorphon, Oxymorphon und pharmazeutisch annehmbare Salze davon.

4. Pharmazeutische Zusammensetzung zur Verwendung gemäß Anspruch 3, wobei besagter Opioid-Antagonist Oxycodonhydrochlorid ist.

5. Pharmazeutische Zusammensetzung zur Verwendung gemäß einem der Ansprüche 1 bis 4, wobei besagte Zusammensetzung Naloxonhydrochlorid und Oxycodonhydrochlorid als die einzigen pharmazeutisch wirksamen Substanzen in einem 1:2-Gewichtsverhältnis umfasst.

6. Pharmazeutische Zusammensetzung zur Verwendung gemäß einem der Ansprüche 1 bis 5, wobei besagte Zusammensetzung eine Darreichungsform mit sofortiger Wirkstofffreisetzung ist.

7. Pharmazeutische Zusammensetzung zur Verwendung gemäß einem der Ansprüche 1 bis 5, wobei besagte Zusammensetzung eine Darreichungsform mit retardierter Wirkstofffreisetzung ist.

8. Pharmazeutische Zusammensetzung zur Verwendung gemäß Anspruch 7, wobei besagte Darreichungsform mit retardierter Wirkstofffreisetzung eine Retard-Matrix oder eine Retard-Beschichtung umfasst.

9. Pharmazeutische Zusammensetzung zur Verwendung gemäß Anspruch 7 oder 8, wobei besagte Darreichungsform Naloxon oder ein pharmazeutisch annehmbares Salz davon umfasst und wobei besagte Darreichungsform Naloxon oder ein pharmazeutisch annehmbares Salz davon mit einem gemäß dem Paddle Test des Europäischen Arzneibuchs gemessenem Tempo freisetzt von:
10 - 30 % Gewicht Naloxon oder besagtem Salz davon in 15 min,
30 - 50 % Gewicht Naloxon oder besagtes Salz davon in 1 h,
45 - 65 % Gewicht Naloxon oder besagtes Salz davon in 2 h,
60 - 85 % Gewicht Naloxon oder besagtes Salz davon in 4 h,
70 - 95 % Gewicht Naloxon oder besagtes Salz davon in 7 h, und
≥ 80 % Gewicht Naloxon oder besagtes Salz davon in 10 h.

10. Orale pharmazeutische Zusammensetzung umfassend mindestens Oxycodon oder ein pharmazeutisch annehmbares Salz davon und Naloxon oder ein pharmazeutisch annehmbares Salz davon zur Verwendung in der Behandlung von Schmerzen in Patienten, die anderenfalls die Opioid-basierte Schmerztherapie wegen der Ausbildung von Harnverhalt beenden müssten, wobei besagte Zusammensetzung eine Darreichungsform mit retardierter Wirkstofffreisetzung ist.

11. Pharmazeutische Zusammensetzung zur Verwendung gemäß Anspruch 10, wobei besagte Zusammensetzung Oxycodonhydrochlorid und Naloxonhydrochlorid umfasst.

12. Pharmazeutische Zusammensetzung zur Verwendung gemäß Anspruch 10 oder 11, wobei besagte Zusammensetzung Oxycodon und Naloxon oder ihre Hydrochloridsalze in einem 2:1-Gewichtsverhältnis umfasst.

13. Pharmazeutische Zusammensetzung zur Verwendung gemäß einem der Ansprüche 10 bis 12, wobei besagte Zusammensetzung Oxycodon oder ein pharmazeutisch annehmbares Salz davon in einer Menge von 5 bis 160 mg pro Einzeldosis und Naloxon oder ein pharmazeutisch annehmbares Salz davon in einer Menge von 2,5 bis 80 mg pro Einzeldosis umfasst.

14. Pharmazeutische Zusammensetzung zur Verwendung gemäß einem der Ansprüche 10 bis 13, wobei besagte Darreichungsform mit retardierter Wirkstofffreisetzung eine Retard-Matrix oder eine Retard-Beschichtung umfasst.

15. Pharmazeutische Zusammensetzung zur Verwendung gemäß Anspruch 14, wobei besagte Darreichungsform Oxycodon oder ein pharmazeutisch annehmbares Salz davon und Naloxon oder ein pharmazeutisch annehmbares Salz davon umfasst und wobei besagte Darreichungsform Oxycodon oder ein pharmazeutisch annehmbares Salz davon und Naloxon oder ein pharmazeutisch annehmbares Salz davon, mit einem gemäß dem Paddle Test des Europäischen Arzneibuchs gemessenem Tempo freisetzt von:
10 - 30 % Gewicht Oxycodon oder besagtes Salz davon in 15 min,
30 - 50 % Gewicht Oxycodon oder besagtes Salz davon in 1 h,
45 - 65 % Gewicht Oxycodon oder besagtes Salz davon in 2 h,
60 - 85 % Gewicht Oxycodon oder besagtes Salz davon in 4 h,
70 - 95 % Gewicht Oxycodon oder besagtes Salz davon in 7 h, und
≥ 80 % Gewicht Oxycodon oder besagtes Salz davon in 10 h, und
10 - 30 % Gewicht Naloxon oder besagtes Salz davon in 15 min,
30 - 50 % Gewicht Naloxon oder besagtes Salz davon in 1 h,
45 - 65 % Gewicht Naloxon oder besagtes Salz davon in 2 h,
60 - 85 % Gewicht Naloxon oder besagtes Salz davon in 4 h,
70 - 95 % Gewicht Naloxon oder besagtes Salz davon in 7 h, und
≥ 80 % Gewicht Naloxon oder besagtes Salz davon in 10 h

## Revendications

1. Composition pharmaceutique comprenant au moins un antagoniste des opiacés ou un sel de celui-ci pharmaceutiquement acceptable et au moins un agoniste des opiacés ou un sel de celui-ci pharmaceutiquement acceptable pour un usage dans le traitement de la rétention urinaire, ledit antagoniste des opiacés étant de la naloxone ou un sel de celle-ci pharmaceutiquement acceptable et ladite composition pharmaceutique étant formulée pour une application orale.

2. Composition pharmaceutique pour un usage selon la revendication 1, dans laquelle ledit antagoniste des opiacés est de l'hydrochlorure de naloxone.

3. Composition pharmaceutique pour un usage selon la revendication 1, dans laquelle ledit agoniste des opiacés est choisi dans le groupe constitué par l'oxycodone, la morphine, l'hydromorphone, l'oxymorphone et les sels de ceux-ci pharmaceutiquement acceptables.

4. Composition pharmaceutique pour un usage selon la revendication 3, dans laquelle ledit agoniste des opiacés est de l'hydrochlorure d'oxycodone.

5. Composition pharmaceutique pour un usage selon l'une quelconque des revendications 1 à 4, dans laquelle ladite composition comprend de l'hydrochlorure de naloxone et de l'hydrochlorure d'oxycodone en tant que seuls agents pharmaceutiquement actifs dans un ratio en poids de 1 : 2.

6. Composition pharmaceutique pour un usage selon l'une quelconque des revendications 1 à 5, dans laquelle ladite composition est une forme posologique à libération immédiate.

7. Composition pharmaceutique pour un usage selon l'une quelconque des revendications 1 à 5, dans laquelle ladite composition est une forme posologique à libération contrôlée.

8. Composition pharmaceutique pour un usage selon la revendication 7, dans laquelle ladite forme posologique à libération contrôlée comprend une matrice à libération contrôlée ou un enrobage à libération contrôlée.

9. Composition pharmaceutique pour un usage selon la revendication 7 ou 8, dans laquelle ladite forme posologique comprend de la naloxone ou un sel de celle-ci pharmaceutiquement acceptable et dans laquelle ladite forme posologique libère la naloxone ou un sel de celle-ci pharmaceutiquement acceptable lors de la mesure selon le test avec un appareil à palette de la Pharmacopée Européenne à un taux :
de 10 à 30 % en poids de naloxone ou dudit sel de celle-ci au bout de 15 minutes,
de 30 à 50 % en poids de naloxone ou dudit sel de celle-ci au bout de 1 heure,
de 45 à 65 % en poids de naloxone ou dudit sel de celle-ci au bout de 2 heures,
de 60 à 85 % en poids de naloxone ou dudit sel de celle-ci au bout de 4 heures,
de 70 à 95 % en poids de naloxone ou dudit sel de celle-ci au bout de 7 heures, et
≥ 80 % en poids de naloxone ou dudit sel de celle-ci au bout de 10 heures.

10. Composition pharmaceutique orale comprenant au moins de l'oxycodone ou un sel pharmaceutiquement acceptable de celui-ci et de la naloxone ou un sel de celle-ci pharmaceutiquement acceptable pour un usage dans le traitement de la douleur chez des patients qui autrement devraient arrêter leur thérapie contre la douleur basée sur les opiacés à cause d'un développement de la rétention urinaire, ladite composition étant une forme posologique à libération contrôlée.

11. Composition pharmaceutique pour un usage selon la revendication 10, dans laquelle ladite composition comprend de l'hydrochlorure d'oxycodone et de l'hydrochlorure de naloxone.

12. Composition pharmaceutique pour un usage selon la revendication 10 ou 11, dans laquelle ladite composition comprend de l'oxycodone et de la naloxone ou leurs sels hydrochlorures dans un ratio en poids de 2 : 1.

13. Composition pharmaceutique pour un usage selon l'une quelconque des revendications 10 à 12, dans laquelle ladite composition comprend de l'oxycodone ou un sel de celui-ci pharmaceutiquement acceptable dans une quantité de 5 à 160 mg par dose unitaire et de la naloxone ou un sel de celle-ci pharmaceutiquement acceptable dans une quantité de 2,5 à 80 mg par dose unitaire.

14. Composition pharmaceutique pour un usage selon l'une quelconque des revendications 10 à 13, dans laquelle ladite forme posologique à libération contrôlée comprend une matrice à libération contrôlée ou un enrobage à libération contrôlée.

15. Composition pharmaceutique pour un usage selon la revendication 14, dans laquelle ladite forme posologique comprend de l'oxycodone ou un sel de celui-ci pharmaceutiquement acceptable et de la naloxone ou un sel de celle-ci pharmaceutiquement acceptable et ladite forme posologique libérant l'oxycodone ou un sel de celui-ci pharmaceutiquement acceptable et la naloxone ou un sel de celle-ci pharmaceutiquement acceptable lors de la mesure selon le test avec un appareil à palette de la Pharmacopée Européenne à un taux :
de 10 à 30 % en poids d'oxycodone ou dudit sel de celui-ci au bout de 15 minutes,
de 30 à 50 % en poids d'oxycodone ou dudit sel de celui-ci au bout de 1 heure,
de 45 à 65 % en poids d'oxycodone ou dudit sel de celui-ci au bout de 2 heures,
de 60 à 85 % en poids d'oxycodone ou dudit sel de celui-ci au bout de 4 heures,
de 70 à 95 % en poids d'oxycodone ou dudit sel de celui-ci au bout de 7 heures, et
≥ 80 % en poids d'oxycodone ou dudit sel de celui-ci au bout de 10 heures et
de 10 à 30 % en poids de naloxone ou dudit sel de celle-ci au bout de 15 minutes,
de 30 à 50 % en poids de naloxone ou dudit sel de celle-ci au bout de 1 heure,
de 45 à 65 % en poids de naloxone ou dudit sel de celle-ci au bout de 2 heures,
de 60 à 85 % en poids de naloxone ou dudit sel de celle-ci au bout de 4 heures,
de 70 à 95 % en poids de naloxone ou dudit sel de celle-ci au bout de 7 heures, et
≥ 80 % en poids de naloxone ou dudit sel de celle-ci au bout de 10 heures.
